(19) Europäisches Patentamt
European Patent Office
Office européen des brevets

(11) EP 3 640 637 A1

(12) # EUROPEAN PATENT APPLICATION

published in accordance with Art. 153(4) EPC

(43) Date of publication:
**22.04.2020 Bulletin 2020/17**

(21) Application number: **18817435.3**

(22) Date of filing: **14.06.2018**

(51) Int Cl.:
***G01N 27/416** (2006.01)* ***G01N 27/26** (2006.01)*
***G01N 27/28** (2006.01)* ***G01N 27/30** (2006.01)*
***G01N 27/49** (2006.01)*

(86) International application number:
**PCT/JP2018/022781**

(87) International publication number:
**WO 2018/230660 (20.12.2018 Gazette 2018/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.06.2017 JP 2017118895**

(71) Applicants:
- **Keio University**
  **Tokyo 108-8345 (JP)**
- **Functional Water Foundation**
  **Tokyo 141-0021 (JP)**

(72) Inventors:
- **EINAGA, Yasuaki**
  **Kanagawa 223-8522 (JP)**
- **WATANABE, Takeshi**
  **Kanagawa 223-8522 (JP)**
- **AKAI, Kazumi**
  **Kanagawa 223-8522 (JP)**
- **NAGASHIMA, Shinichi**
  **Kanagawa 223-8522 (JP)**
- **KODAMA, Tomoko**
  **Kanagawa 223-8522 (JP)**
- **HOTTA, Kunimoto**
  **Tokyo 141-0021 (JP)**

(74) Representative: **D Young & Co LLP**
**120 Holborn**
**London EC1N 2DY (GB)**

(54) **RESIDUAL CHLORINE MEASURING METHOD AND RESIDUAL CHLORINE MEASURING APPARATUS**

(57) Provided are a method and apparatus for measuring free residual chlorine concentration that is accurate and simple, which can obtain objective measurement results without using any harmful reagents, and without being affected by the potential window. Provided are a method and apparatus for measuring residual chlorine, in which a conductive diamond electrode is used; and a current value is measured when the potential of the conductive diamond electrode against a silver/silver chloride electrode is set to a potential in the range of from 0 V to +1.6 V and the residual chlorine concentration based on hypochlorite ion is calculated, and a current value is measured when the potential of the conductive diamond electrode against a silver/silver chloride electrode is set to a potential in the range of from +0.4 V to -1.0 V and the residual chlorine concentration based on hypochlorous acid is calculated; and the calculated residual chlorine concentration based on hypochlorite ion and the calculated residual chlorine concentration based on hypochlorous acid are added to make the total residual chlorine concentration. Further provided are an apparatus and method comprising a temperature measuring unit and/or a pH measuring unit.

Fig. 6

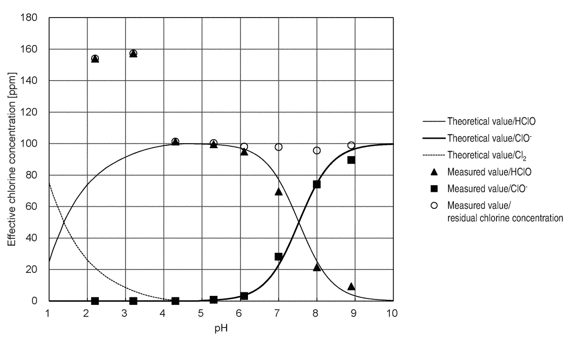


EP 3 640 637 A1

## Description

Technical Field

The present invention relates to methods and apparatuses using an electrochemical method for measuring residual chlorine concentration.

Background Art

Conventionally, methods for measuring residual chlorine in sample solutions include colorimetric methods such as the DPD method, and polarography using electrodes. These measuring methods are described in detail in [Method for examining free residual chlorine and combined residual chlorine based on Clause 2 of Article 17 of Ordinance for Enforcement of Water Supply Act](The Ministry of Health, Labor and Welfare Notification No. 318 (Sept. 29, 2003)).

For example, the DPD method is a method for measuring residual chlorine concentration by colorimetric comparison of a peach-red color developed by the reaction of residual chlorine in a sample solution with diethyl-p-phenylenediamine (DPD), with a standard colorimetric table by a measurement operator. This method has problems such as high costs of the reagent; the possibility that individual differences could arise in the measurement results; treatment of the waste liquid after measurement is necessary; and the residual chlorine concentration cannot be measured continuously since sample collection is needed in the measurement.

The polarography method using electrodes is a method for measuring residual chlorine concentration by measuring current values flowing through a working electrode. This method does not require any reagents, individual differences do not arise and waste liquid treatment after the measurement is unnecessary. However, conventional polarography methods use a platinum electrode for the working electrode as described in Patent Literature 1, and, therefore, are problematic in that the oxidation current peak of residual chlorine appears only in the vicinity of the limit of the potential window and overlaps with (hangs in) the potential window thereby inhibiting accurate measurement.

Patent Literature 2 describes a technique in which a voltammetric measurement using a three-electrode system is carried out by using a boron-doped conductive diamond electrode as the working electrode in place of a platinum electrode and combining the electrode with a counter electrode and a reference electrode. According to this technique, no reagent is needed; objective measurement results can be obtained; and the residual chlorine concentration measurement can be carried out accurately and readily without being influenced by the potential window. However, this technique may have difficulty measuring the residual chlorine concentration depending on the pH of the sample solution. More specifically, when the pH of a sample solution falls below 6 (becomes acidic) and the measured current value decreases, the measurement may become difficult.

Patent Literature 3 describes a method for measuring concentrations of ozone, hypochlorous acid, hypochlorite ion, chlorine and hydrogen peroxide contained in a solution. According to Patent Literature 3, in order to measure concentrations of hypochlorous acid, hypochlorite ion and chlorine, first, pH measurement of a sample solution is carried out. Then, if the pH is 4 or lower, the reduction current value of hypochlorous acid is measured by a cyclic voltammetry using a gold microelectrode without adjusting the pH of the sample solution and the concentration of hypochlorous acid is calculated. Next, numerical values of the measured pH and hypochlorous acid concentration are applied to a graph showing the abundance ratios (presence ratios) of hypochlorous acid (HClO), hypochlorite ion ($ClO^-$) and chlorine ($Cl_2$) depending on the pH (see, for example, Figure 1 of the present specification), and the amount of generated chlorine is determined by calculation. Incidentally, Figure 1 of the present specification is a graph made by graphing the abundance ratios of hypochlorous acid (HClO), hypochlorite ion ($ClO^-$) and chlorine ($Cl_2$) based on "Figure 3.1 pH and compositional (presence) ratio of effective chlorine" shown on page 73 of Masaki Matsuo, "Fundamentals and Utilization Technologies of Electrolytic Water", 1st edition, 1st printing, Gihodo Shuppan Co., Ltd. (in Japanese) (Non Patent Literature 1).

In the method disclosed in Patent Literature 3, when the measured pH of a sample solution is 4 to 5.5, the reduction current value of hypochlorous acid is measured by cyclic voltammetry using a gold microelectrode without adjusting the pH and the concentration of hypochlorous acid is calculated.

In the method described in Patent Literature 3, when the measured pH is 5.5 to 8.9, cyclic voltammetry using a gold microelectrode cannot accurately determine the reduction current value of hypochlorous acid or hypochlorite ion. As such, the pH of the sample solution is adjusted to be more acidic (pH: 5.5 or lower) or more alkaline (pH: 8.9 or higher) by using HCl, NaOH or the like, and then the reduction current value of hypochlorous acid or hypochlorite ion is measured.

In the method described in Patent Literature 3, when the measured pH of a sample solution is 8.9 or higher, the reduction current of hypochlorite ion is measured by cyclic voltammetry using a gold microelectrode without adjusting the pH of the sample solution and the concentration of hypochlorite ion is calculated. Summarizing the above, the method disclosed in Patent Literature 3 can be described as follows.
$pH < 4$

The concentration of hypochlorous acid is measured and the amount of generated chlorine is estimated from Figure 1.
4 < pH < 5.5
The concentration of hypochlorous acid is measured.
5.5 < pH < 8.9
The sample solution is adjusted to be at a pH of 5.5 or lower or at a pH of 8.9 or higher, and the concentration of hypochlorous acid or hypochlorite ion is measured.
8.9 < pH
The concentration of hypochlorite ion is measured.

According to the procedure presented in Patent Literature 3, the concentrations of ozone, hypochlorous acid, hypochlorite ion, chlorine and hydrogen peroxide can be measured by combining pH measurement, electrochemical measurement and spectroscopic measurement of a sample solution. In this procedure, however, it is necessary to know the pH of the sample solution in advance of the measurement of concentrations of hypochlorous acid, hypochlorite ion and chlorine, and moreover, depending on the result of the measured pH value, it is necessary to adjust the pH of the sample solution before measurement of the residual chlorine concentration; thus, the procedure cannot necessarily be regarded as a simple method.

Citation List

Patent Literature

Patent Literature 1: JP Patent Publication (Kokoku) No. 55-017939
Patent Literature 2: JP Patent Publication (Kokai) No. 2007-139725 (JP Patent No. 4734097)
Patent Literature 3: JP Patent Publication (Kokai) No. 2003-240712

Non Patent Literature

Non Patent Literature 1: Masaki Matsuo, "Fundamentals and Utilization Technologies of Electrolytic Water", 1st edition, 1st printing, published by Gihodo Shuppan Co., Ltd. (Jan. 25, 2000) (in Japanese)

Summary of Invention

Technical Problem

It is an object of the present invention (disclosure) to provide means and an apparatus for measuring free residual chlorine concentration, to solve the conventional problems. Further, it is an object of the present invention to provide means and an apparatus for calculating the pH of a sample solution, utilizing measurement results of residual chlorine concentration. Further, when the pH of a sample solution is known, it is an object of the present invention to provide means and an apparatus for measuring residual chlorine concentration, which can be used to carry out measurement over a broad pH range by adopting voltammetric measurement conditions using a three-electrode system adapted for the pH of the sample solution.

Further, the present inventors have found that results of chlorine concentration measurement by a spectrophotometer varies largely depending on the temperature of the solution (Figure 12). Therefore, in one embodiment, it is an object of the present invention to provide an apparatus capable of accurately measuring chlorine concentration even when the solution temperature varies.

Solution to Problem

In order to solve the problem(s) above, the present invention (disclosure) provides an apparatus comprising a conductive diamond electrode and method, for the measurement of residual chlorine concentration. By carrying out an electrochemical measurement using this method or apparatus, the residual chlorine concentration in an aqueous solution can be measured simply and accurately. Further, in order to solve the problem(s) above, the present invention provides an apparatus comprising a temperature measuring unit and/or a pH measuring unit and method.

That is, the present invention (disclosure) encompasses the following.

[1] A method for measuring the residual chlorine concentration in a sample solution possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and the reference electrode is a silver/silver chloride electrode,
wherein the method comprises:

(i) measuring the current value when the potential of the conductive diamond electrode against the silver/silver chloride electrode is set to a given potential in the range of from 0 V to +1.6 V and calculating the residual chlorine concentration based on hypochlorite ion;
(ii) measuring the current value when the potential of the conductive diamond electrode against the silver/silver chloride electrode is set to a given potential in the range of from +0.4 V to -1.0 V and calculating the residual chlorine concentration based on hypochlorous acid; and
(iii) adding the residual chlorine concentration based on hypochlorite ion calculated in step (i) above and the residual chlorine concentration based on hypochlorous acid calculated in step (ii) above, designating said total residual chlorine concentration obtained by the addition as the residual chlorine concentration of the sample solution.

[2] The method of 1, further comprising, after step (iii),

(iv) bringing a temperature measuring unit into contact with the sample solution and measuring the solution temperature of the sample solution with said temperature measuring unit, and calculating the temperature correction value from the measured solution temperature; and
(v) carrying out correction on said total residual chlorine concentration obtained by the addition according to 1 based on the temperature correction value in step (iv), and designating the total residual chlorine concentration after the correction as the residual chlorine concentration of the sample solution.

[3] A method for measuring the pH of a sample solution possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and the reference electrode is a silver/silver chloride electrode,
wherein the method comprises:

(i) measuring the current value when the potential of the conductive diamond electrode against the silver/silver chloride electrode is set to a given potential in the range of from 0 V to +1.6 V and calculating the residual chlorine concentration based on hypochlorite ion;
(ii) measuring the current value when the potential of the conductive diamond electrode against the silver/silver chloride electrode is set to a given potential in the range of from +0.4 V to -1.0 V and calculating the residual chlorine concentration based on hypochlorous acid;
(iii) calculating the compositional ratio of hypochlorite ion and hypochlorous acid by comparing the residual chlorine concentration based on hypochlorite ion calculated in step (i) and the residual chlorine concentration based on hypochlorous acid calculated in step (ii); and
(iv) calculating pH by applying the calculated compositional ratio to an effective chlorine compositional ratio curve, designating said calculated pH as the pH of the sample solution.

[4] The method of 3, further comprising, after step (iv),

(v) bringing a temperature measuring unit into contact with the sample solution and measuring the solution temperature of the sample solution with said temperature measuring unit, and calculating the temperature correction value from the measured solution temperature; and
(vi) carrying out correction on the calculated pH according to 3 based on the temperature correction value in step (v), designating said pH of the sample solution after the correction as the pH of the sample solution.

[5] A method for automatic diagnosis of a measuring instrument, further comprising, after step (iv),

(v) bringing a pH measuring unit into contact with the sample solution, and measuring the pH of the sample solution by the pH measuring unit; and

(vi) comparing the pH calculated from the value of current flowing through the working electrode according to 3 with the pH measured by the pH measuring unit in step (v), wherein when the difference therebetween is within a predetermined error, the measuring instrument is determined to be normal.

[6] The method according to any one of 1 to 5, further comprising an electrode initializing step, wherein the electrode initializing step comprises:
repeating the following steps (i) and (ii) as a pair one or more times:

(i) applying a positive or negative first pulse voltage for 0.01 to 60 sec; and
(ii) applying a negative or positive second pulse voltage, said second pulse voltage having a sign reverse to the pulse voltage applied in step (i), for 0.01 to 60 sec.

[7] A method for measuring the residual chlorine concentration in a sample solution having a known pH and possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein, when the pH of the sample solution is 7.5 or lower, the method comprises: measuring the current value when the potential of the conductive diamond electrode against the silver/silver chloride electrode is set to a given potential in the range of from +0.4 V to -1.0 V and calculating the concentration of hypochlorous acid; and
designating the residual chlorine concentration calculated by applying the pH of the sample solution and the calculated hypochlorous acid concentration to an effective chlorine compositional ratio curve, as the residual chlorine concentration of the sample solution.
[8] The method of 7, wherein the pH of the sample solution is 4 to 7.5.
[9] A method for measuring the residual chlorine concentration in a sample solution having a known pH and possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein, when the pH of the sample solution is 7.5 or higher, the method comprises: measuring a current value when the potential of the conductive diamond electrode against the silver/silver chloride electrode is set to a given potential in the range of from 0 V to +1.6 V and calculating the concentration of hypochlorite ion; and
designating the residual chlorine concentration calculated by applying the pH of the sample solution and the calculated hypochlorite ion concentration to an effective chlorine compositional ratio curve, as the residual chlorine concentration of the sample solution.
[10] The method of 9, wherein the pH of the sample solution is higher than 7.5 and 10 or lower.
[11] The method according to any one of 1 to 10, wherein the measurement is carried out continuously by a flow injection method.
[12] The method of 11, wherein the measurement is carried out at a constant potential.
[13] A continuous measuring method, comprising repeating the following steps (a) and (b) as a pair one or more times:

(a) carrying out the electrode initializing step according to 6 before measurement; and then
(b) carrying out the constant-potential measurement according to 12.

[14] A residual chlorine measurement apparatus for measuring the residual chlorine concentration in a sample solution, said apparatus comprising:
a working electrode; a counter electrode; a reference electrode; a voltage applying unit for applying a voltage between the working electrode and the reference electrode; a current measuring unit for measuring the value of current flowing through the working electrode under the applied voltage; and an information processing device for calculating the residual chlorine concentration based on a current measurement signal from the current measuring unit,
wherein the working electrode is a boron doped conductive diamond electrode;
the reference electrode is a silver/silver chloride electrode; and
the information processing device

(i) measures the current value by controlling the potential of the conductive diamond electrode against the silver/silver chloride electrode at a given potential in the range of from 0 V to +1.6 V;

(ii) measures the current value by controlling the potential of the conductive diamond electrode against the silver/silver chloride electrode at a given potential in the range of from +0.4 V to -1.0 V; and
(iii) calculates the residual chlorine concentration based on hypochlorite ion from the current value measured in step (i), calculates the residual chlorine concentration based on hypochlorous acid from the current value measured in step (ii), designating the total residual chlorine concentration obtained by adding the calculated residual chlorine concentration based on hypochlorite ion and the calculated residual chlorine concentration based on hypochlorous acid, as the residual chlorine concentration of the sample solution,

wherein the measurement in step (i) and the measurement in step (ii) can be carried out successively in any order, or simultaneously.
[15] The apparatus of 14, further comprising: a temperature measuring unit for measuring the temperature of the sample solution; and a second information processing device for calculating the temperature of the sample solution based on the temperature measurement signal from the temperature measuring unit, wherein, after step (iii), the apparatus

(iv) brings the temperature measuring unit into contact with the sample solution, measures the solution temperature of the sample solution with said temperature measuring unit, and calculates a temperature correction value from the measured solution temperature; and
(v) carries out correction on the total residual chlorine concentration obtained by the addition according to 14 based on the temperature correction value in step (iv), and designates the total residual chlorine concentration after the correction as the residual chlorine concentration of the sample solution.

[16] An apparatus for measuring the pH of a sample solution possibly containing residual chlorine, said apparatus comprising:
a working electrode; a counter electrode; a reference electrode; a voltage applying unit for applying a voltage between the working electrode and the reference electrode; a current measuring unit for measuring the value of current flowing through the working electrode under the applied voltage; and an information processing device for calculating the residual chlorine concentration based on a current measurement signal from the current measuring unit,
wherein the working electrode is a boron doped conductive diamond electrode;
the reference electrode is a silver/silver chloride electrode; and
wherein the information processing device

(i) measures the current value by controlling the potential of the conductive diamond electrode against the silver/silver chloride electrode at a given potential in the range of from 0 V to +1.6 V;
(ii) measures the current value by controlling the potential of the conductive diamond electrode against the silver/silver chloride electrode at a given potential in the range of from +0.4 V to -1.0 V; and
(iii) calculates the residual chlorine concentration based on hypochlorite ion from the current value measured in step (i), calculates the residual chlorine concentration based on hypochlorous acid from the current value measured in step (ii), and calculating the compositional ratio of hypochlorite ion and hypochlorous acid by comparing the residual chlorine concentration calculated in step (i) and the residual chlorine concentration calculated in step (ii); and
(iv) calculates a pH by applying the calculated compositional ratio to an effective chlorine compositional ratio curve, and designates the calculated pH as the pH of the sample solution, wherein the measurement in step (i) and the measurement in step (ii) can be carried out successively in any order, or simultaneously.

[17] The apparatus of 16, further comprising: a temperature measuring unit for measuring the temperature of the sample solution; and a second information processing device for calculating the temperature of the sample solution based on the temperature measurement signal from the temperature measuring unit, wherein, after step (iv), the apparatus

(v) brings the temperature measuring unit into contact with the sample solution, measures the solution temperature of the sample solution with said temperature measuring unit, and calculates a temperature correction value from the measured solution temperature; and
(vi) carries out correction on the calculated pH according to 16 based on the temperature correction value in step (v), and designates the pH of the sample solution after the correction as the pH of the sample solution.

[18] The apparatus of 16, further comprising: a pH measuring unit for measuring the pH of a sample solution; and a second information processing device for calculating the pH of the sample solution based on a pH measurement signal from the pH measuring unit, wherein the apparatus further comprises an automatically diagnosing function of the following (v) and (vi) after step (iv),

(v) bringing the pH measuring unit into contact with the sample solution, and measuring the pH of the sample solution by the pH measuring unit; and
(vi) comparing the pH calculated from the value of current flowing through the working electrode according to 16 with the pH measured by the pH measuring unit in step (v), wherein when the difference therebetween is within a predetermined error, the measuring instrument is determined to be normal.

[19] The apparatus of any one of 14 to 18, comprising the temperature measuring unit of 15 or 17, and the pH measuring unit of 18.
[20] The apparatus according to any one of 14 to 19, comprising a bipotentiostat and two working electrodes, wherein the measurement in step (i) and the measurement in step (ii) can be carried out simultaneously.
[21] The apparatus according to any one of 14 to 19, comprising two working electrodes, two counter electrodes and two reference electrodes, wherein the measurement in step (i) and the measurement in step (ii) can be carried out simultaneously.
[22] The apparatus according to any one of 14 to 21 for flow injection analysis, further comprising a flow cell, wherein the flow cell comprises the working electrode(s), reference electrode(s) and counter electrode(s) built-in, and comprises a flow tube for passing the sample solution, wherein the working electrode(s), the reference electrode(s) and the counter electrode(s) are arranged in the flow cell such that when the sample solution passes through the flow tube in the flow cell, the sample solution can contact with the working electrode(s), the reference electrode(s) and the counter electrode(s).
[23] The apparatus of 22, wherein the flow cell further comprises a temperature measuring unit and/or pH measuring unit built-in; and the working electrode(s), the reference electrode(s) and the counter electrode(s), and the temperature measuring unit and/or the pH measuring unit are arranged in the flow cell such that when the sample solution passes through the flow tube in the flow cell, the sample solution can further contact with the temperature measuring unit and/or the pH measuring unit.
[24] The apparatus according to any one of 14 to 23, wherein the reference electrode(s) is a silver electrode.
[25] The apparatus according to any one of 14 to 24, wherein the counter electrode(s) is a boron doped conductive diamond electrode.
[26] The apparatus according to any one of 14 to 25, wherein the apparatus further carries out, as an electrode initializing step, said electrode initialization step comprising:
repeating the following steps (i) and (ii) as a pair one or more times:

(i) applying a positive or negative first pulse voltage for 0.01 to 60 sec; and
(ii) applying a negative or positive second pulse voltage, said second pulse voltage having a sign reverse to the pulse voltage applied in step (i), for 0.01 to 60 sec.

The present specification includes the contents of the disclosure of Japanese Patent Application No. 2017-118895, based on which priority of the present application is claimed.

Advantageous Effects of Invention

According to the present invention (disclosure), it is possible to provide means and an apparatus for measuring free residual chlorine concentration, which can obtain objective measurement results without using any harmful reagents, without being affected by the potential window and without the need to measure the pH of a sample solution in advance. Further, according to the present invention (disclosure), by comparing the reduction-side residual chlorine concentration with the oxidation-side residual chlorine concentration the pH of a sample solution can be measured at the same time.
Further, when the pH of a sample solution is known, by adopting voltammetric measurement conditions using a three-electrode system adjusted to the pH of the sample solution, the residual chlorine concentration can be measured over a broad pH range without any pH region where measurement is difficult. Further, in one embodiment, even when the temperature of the solution varies, the residual chlorine concentration can be accurately measured by temperature correction.

Brief Description of Drawings

[Figure 1] Figure 1 shows the compositional ratio of effective chlorine (available chlorine).

[Figure 2-1] Figure 2-1 is a schematic constitution diagram of the residual chlorine concentration measuring apparatus according to the first embodiment of the present invention.

[Figure 2-2] Figure 2-2 is a constitution diagram illustrating a modified example of the first embodiment comprising a temperature measuring unit and a pH measuring unit.

[Figure 3] Figure 3 shows voltammograms indicating residual chlorine at each pH when the potential of the working electrode is swept from +0.4 V to +2.0 V in the embodiment above.

[Figure 4] Figure 4 shows voltammograms indicating residual chlorine at each pH when the potential of the working electrode is swept from +0.4 V to -1.6 V in the embodiment above.

[Figure 5] Figure 5 shows voltammograms collectively showing the results of Figure 3 and Figure 4.

[Figure 6] Figure 6 shows measurement results of the residual chlorine concentration. This shows the effective chlorine concentration.

[Figure 7] Figure 7 shows oxidation-side voltammograms.

[Figure 8] Figure 8 shows an oxidation-side calibration curve.

[Figure 9] Figure 9 shows reduction-side voltammograms.

[Figure 10] Figure 10 shows a reduction-side calibration curve.

[Figure 11-1] Figure 11-1 is a schematic constitution diagram of the residual chlorine concentration measuring apparatus according to the second embodiment of the present invention.

[Figure 11-2] Figure 11-2 is a constitution diagram illustrating a modified example of the second embodiment comprising a temperature measuring unit and a pH measuring unit.

[Figure 12] Figure 12 shows the dependency of the readout of the absorptiometer (absorption photometer) on the solution temperature (evaluation of the same solutions). Square symbols and round symbols each indicate results for lots measured at different dates and hours, respectively. Both results exhibited a similar linear tendency.

[Figure 13] Figure 13 shows the temperature dependency of the current sensitivity.

[Figure 14] Figure 14 shows reduction currents of solutions measured using a flow cell.

[Figure 15] Figure 15 shows oxidation currents of the solutions measured using a flow cell.

[Figure 16] Figure 16 shows concentration-temperature calibration curves (after 1 sec, salt bridge).

[Figure 17] Figure 17 shows the temperature dependency of the current sensitivity. The gradient of the straight line is the same as that in Figure 13.

[Figure 18] Figure 18 shows concentration-temperature calibration curves (after 1 sec, salt bridge). Plotted data are the same as those in Figure 16.

[Figure 19] Figure 19 shows an example of electrode initializing pulses. (a) are initializing pulses; (b) are reduction measurements; and (c) are oxidation measurements.

[Figure 20] Figure 20 shows measurement results using a silver electrode as the reference electrode.

[Figure 21-1] Figure 21-1 shows the temperature dependency of the effective chlorine compositional ratio.

[Figure 21-2] Figure 21-2 is an enlarged diagram of a part of Figure 21-1.

[Figure 22] Figure 22 shows voltammograms indicating the effect of the electrode initialization.

[Figure 23] In Figure 23, A shows comparison of heat capacities of temperature sensors. B shows sizes of the platinum temperature sensor and the thermocouple that were used.

## Description of Embodiments

Here, the present invention will be described by referencing the drawings.

### <Definitions>

"Effective chlorine (available chlorine)" and "residual chlorine" are defined generally as follows.

"Effective chlorine": a collective term of chlorine-containing chemical species having the germicidal disinfecting action

"Residual chlorine": chlorine remaining in water and sustainingly exhibiting germicidal effect

In the present specification, residual chlorine and effective chlorine are used as mutually interchangeable synonyms, since the residual chlorine indicates all effective chlorine remaining in an aqueous solution.

Residual chlorine is composed of two types of chlorine, namely free residual chlorine and bound residual chlorine:

free residual chlorine is composed of hypochlorous acid (HClO), hypochlorite ion ($ClO^-$) and dissolved chlorine ($CL_{2(aq)}$) (here, aq is an abbreviation of aqueous solution); and

bound residual chlorine is composed of monochloramine ($NH_2Cl$), dichloramine ($NHCl_2$), trichloramine ($NCl_3$) and the like.

Various types of chloramines are formed when a substance having an =NH group such as ammonia or the like is present in water and the same reacts with chlorine. Chloride ion ($Cl^-$) does not have germicidal action and, therefore, is not included in residual chlorine (effective chlorine) by definition.

The above descriptions can be summarized as follows.

$$[\text{Residual chlorine (effective chlorine)}] = [\text{free residual chlorine } (HClO + ClO^- + CL_{2(aq)}] + [\text{bound residual chlorine (chloramines and the like)}$$

In the present specification, unless specified otherwise, free residual chlorine concentration is described as residual chlorine concentration.

<The residual chlorine measuring method according to the present invention>

In one embodiment, the present invention (disclosure) provides a method for measuring residual chlorine comprising bringing a working electrode, a counter electrode and a reference electrode into contact with a sample solution possibly containing residual chlorine, which is the object being measured, applying a voltage between the working electrode and the reference electrode, and measuring the value of current flowing through the working electrode under the voltage, and thereby calculating the concentration of the residual chlorine. In one embodiment, the working electrode is a boron doped conductive diamond electrode, and the reference electrode is a silver/silver chloride electrode. In this method, the total residual chlorine concentration is measured by adding the numerical values of the residual chlorine concentration obtained from a current measurement value when the potential of the conductive diamond electrode against the reference electrode is set to a given potential in the range of from 0 V to +1.6 V (in the present specification, this may be referred to as the oxidation-side residual chlorine concentration, or the residual chlorine concentration based on hypochlorite ion), and the residual chlorine concentration obtained from a current measurement value when the potential of the conductive diamond electrode against the reference electrode is set to a given potential in the range of from 0.4 V to -1.0 V (in the present specification, this may be referred to as the reduction-side residual chlorine concentration, or the residual chlorine concentration based on hypochlorous acid).

Here, in order to measure the oxidation-side residual chlorine concentration, the reason for setting the potential of the conductive diamond electrode against the reference electrode to from 0 V to +1.6 V is because the peak of the current caused by an oxidation reaction of hypochlorite ion (hereinafter, referred to as oxidation current) appears between 0 V and +1.6 V, and at a potential lower than 0 V, oxidation reaction of hypochlorite ion does not occur. The potential for measuring the oxidation-side residual chlorine concentration can be set to a given potential in the range of from 0 V to +1.6 V, and can be made to be, for example, 0 V or higher, +0.1 V or higher, +0.2 V or higher, +0.3 V or higher, +0.4 V or higher, +0.5 V or higher, +0.6 V or higher, +0.7 V or higher, +0.8 V or higher, +0.9 V or higher, +1.0 V or higher or +1.1 V or higher, and +1.6 V or lower, +1.5 V or lower, +1.45 V or lower or +1.4 V or lower, and can be set to a potential in a range in any combination of these.

Further, in order to measure the reduction-side residual chlorine concentration, the reason for setting the potential of the conductive diamond electrode against the reference electrode to from 0.4 V to -1.0 V is because the peak of the current caused by a reduction reaction of hypochlorous acid and a reduction reaction of dissolved chlorine (hereinafter, referred to as reduction current) appears between 0.4 V and -1.0 V, and at 0.4 V or higher, almost no reduction reactions of hypochlorous acid and dissolved chlorine occur. The potential for measuring the reduction-side residual chlorine concentration can be set to a given potential in the range of from +0.4 V to -1.0 V, and can be made to be, for example, -1.0 V or higher, -0.9 V or higher or -0.8 V or higher, and +0.4 V or lower, +0.3 V or lower, +0.2 V or lower, +0.1 V or lower, 0 V or lower, -0.1 V or lower, -0.2 V or lower, -0.3 V or lower or -0.4 V or lower, and can be set to a potential in a range in any combination of these.

In the embodiment above, it is not necessary to measure the pH of the sample solution in advance in order to measure residual chlorine, and the reason for this is described below.

When the pH of the sample solution is 5 or lower, it is believed that hypochlorite ion does not exist according to the compositional ratio curves of effective chlorine of Figure 1. Therefore, no oxidation current occurs and the reduction current indicates the residual chlorine concentration. Incidentally, when the pH of a sample solution is 4 to 5, the abundance ratio of dissolved chlorine is low according to the compositional ratio curves of effective chlorine in Figure 1 and hypochlorous acid is predominantly present. As such, it is believed that measurement error due to dissolved chlorine is minimal, and the residual chlorine concentration can be measured based on the response current by the reduction reaction.

When the pH of the sample solution is 5 to 10, dissolved chlorine does not exist according to the compositional ratio curves of effective chlorine in Figure 1, and hypochlorous acid and hypochlorite ion are present. Therefore, the total residual chlorine concentration can be measured by adding the reduction-side residual chlorine concentration obtained from the reduction current and the oxidation-side residual chlorine concentration obtained from the oxidation current.

When the pH of the sample solution is 10 or higher, it is believed that dissolved chlorine and hypochlorous acid do not exist according to the compositional ratio curves of effective chlorine in Figure 1. Therefore, no reduction current occurs and the oxidation current indicates the residual chlorine concentration.

From the above, regardless of whether the pH of the sample solution is 5 or lower (for example, 4 to 5), 5 to 10 or 10 or higher, residual chlorine can be measured, and it is not necessary to measure the pH of the sample solution in advance in order to measure residual chlorine.

<The pH measuring method according to the present invention>

Rather, the residual chlorine measuring method according to the present invention can be utilized and the pH of a sample solution can be measured as follows. That is, in one embodiment, the present invention provides a method for measuring the pH of the sample solution.

1. When only a reduction current is observed:
The pH of the sample solution can be specified to be 5 or lower from the compositional ratio curves of effective chlorine in Figure 1.
2. When a reduction current and an oxidation current are observed:
In this case, the pH can be specified to be 5 < pH < 10; and the pH of the sample solution can be calculated from the compositional ratio curves of effective chlorine in Figure 1 by using the compositional ratio of the reduction-side residual chlorine concentration (residual chlorine concentration based on hypochlorite ion) and the oxidation-side residual chlorine concentration (residual chlorine concentration based on hypochlorous acid). For example, when the reduction-side residual chlorine concentration = (is equal to) the oxidation-side residual chlorine concentration, it can be understood that the pH of the solution = 7.5.
3. When only a oxidation current is observed:
The pH of the sample solution can be specified to be 10 or higher from the compositional ratio curves of effective chlorine in Figure 1.

In one embodiment, the pH measuring method according to the present invention can further comprise carrying out temperature correction. That is, after the pH is measured by the method above, as means separate therefrom, a temperature measuring unit can be brought into contact with the sample solution; the temperature of the sample solution can be measured; and a temperature correction value can be calculated from the measured solution temperature. Then, with regard to the pH calculated by the pH measuring method above (pH calculated from the current value flowing through the working electrode), a temperature correction can be made based on the temperature correction value. The temperature measuring unit will be described later.

In one embodiment, the pH measuring method according to the present invention can further be utilized for automatic diagnosis of a measuring instrument. That is, after the pH is measured by the method above, as means separate therefrom, a pH measuring unit can be brought into contact with the sample solution; the pH of the sample solution can be separately measured by the pH measuring unit; and the pH calculated by the pH measuring method above (pH calculated from a value of current flowing through the working electrode) can be compared with the pH measured by the pH measuring unit. As a result of the comparison, if the difference between the two pHs is within a predetermined error, the measuring instrument can be determined to be normal. The pH measuring unit will be described later.

<The residual chlorine concentration measuring method according to the present invention for a sample solution having a known pH>

In one embodiment, the residual chlorine concentration measuring method according to the present invention can be carried out on a sample solution having a known pH. In cases where the pH of the sample solution is known, by measuring either the concentration of hypochlorite ion or the concentration of hypochlorous acid depending on the pH of the sample solution, the residual chlorine concentration can be measured including the region of pH 6 or lower where measurement was difficult. The pH can also be retrieved by the pH measuring unit.

In this embodiment, the following measurements are carried out depending on the known pH.

1. Cases where the pH of a sample solution is measured and lower than 4, or when the pH of a sample solution having a known pH is known to be lower than 4:
A measurement different from the measurement in the case of a pH of 4 to 10 may be necessary since a measurement error may occur in the reduction current due to the effect of dissolved chlorine because of chemical change of hypochlorous acid. Examples of different residual chlorine concentration measuring methods include, for example, absorbance analysis. In this case, a calibration curve is generated using the actual sample solution by measuring the reduction current under conditions of said pH value and then the residual chlorine concentration measurement is carried out.

2-1. Cases where the pH of a sample solution is 4 to 7.5
The concentration of hypochlorous acid having a high compositional ratio is measured and by using the measurement result and the pH information, the measurement value can be converted to residual chlorine concentration based on the compositional ratio curves of effective chlorine in Figure 1. The error is smaller than that when converting from the measurement value of the hypochlorite ion concentration. For example, when the pH of the sample solution is 7 and the concentration of hypochlorous acid according to the reduction current measurement is 50 ppm, since the compositional ratio of hypochlorous acid is 77%, the residual chlorine concentration is 50 ppm/0.77 = 64.9 ppm.
When the pH of the sample solution is 4 to 7.5, in one embodiment, the concentration of hypochlorite ion need not be measured. By doing so, the measuring time can be shortened.
When the pH of the sample solution is 4 to 7.5, in one embodiment, the concentration of hypochlorite ion may also be optionally measured. By doing so, the measurement precision can be increased.
In one embodiment, the residual chlorine measuring method of the present invention can be carried out on a sample solution having a pH of 7.5 or lower, for example, 7.4 or lower, 7.3 or lower, 7.2 or lower or 7.1 or lower, or for example, 7.0 or lower, 6.9 or lower, 6.8 or lower, 6.7 or lower, 6.6 or lower or 6.5 or lower. In one embodiment, the residual chlorine measuring method of the present invention can be carried out on a sample solution having a pH of 4 or higher, for example, 4.1 or higher, 4.2 or higher, 4.3 or higher, 4.4 or higher, 4.5 or higher, 4.6 or higher, 4.7 or higher, 4.8 or higher or 4.9 or higher, or for example, 5.0 or higher.

2-2. Cases where the pH of a sample solution is 7.5 to 10:
The concentration of hypochlorite ion having a high compositional ratio is measured and by using the measurement result and pH information, the measurement value can be converted to residual chlorine concentration by the compositional ratio curves of effective chlorine in Figure 1. The error is smaller than that when converting from the measurement value of the hypochlorous acid concentration. For example, when the pH of the sample solution is 8, and the concentration of hypochlorite ion according to the oxidation current measurement is 50 ppm, since the compositional ratio of hypochlorite ion is 75%, the residual chlorine concentration is 50 ppm/0.75 = 66.7 ppm.
When the pH of the sample solution is 7.5 to 10, in one embodiment, the concentration of hypochlorous acid is not measured. By doing so, the measuring time can thereby be shortened.
When the pH of the sample solution is 7.5 to 10, in one embodiment, the concentration of hypochlorous acid may also be optionally measured. By doing so, the measurement precision can be increased.
In one embodiment, the residual chlorine measuring method of the present invention can be carried out on a sample solution having a pH of 7.5 or higher. Further, in one embodiment, the residual chlorine measuring method of the present invention can be carried out on a sample solution having a pH of higher than 7.5. In one embodiment, the residual chlorine measuring method of the present invention can be carried out on a sample solution having a pH of 10 or lower, for example, 9.5 or lower, 9 or lower, 8.5 or lower or 8 or lower.

3. Cases where the pH of a sample solution is measured and is higher than 10, or when the pH of a sample solution having a known pH is known to be higher than 10:
Since $OH^-$ imposes a measurement error on the oxidation current, a measurement different from the measurement in the case of a pH of 4 to 10 may be necessary. Examples of different residual chlorine concentration measuring methods include, for example, absorbance analysis. In this case, a calibration curve is generated using the actual sample solution by measuring the oxidation current under conditions of said pH value and then the residual chlorine concentration measurement is carried out.

<First embodiment>

Here, the first embodiment of the present invention will be described by referencing the drawings.

In the first embodiment, the present invention provides a residual chlorine measurement apparatus 100. The residual chlorine measurement apparatus 100 is a batch-type electrochemical measuring apparatus which carries out voltammetric measurement by a three-electrode system, wherein said apparatus carries out analysis of a sample solution by dissolving an electrolyte into the sample solution to make an electrolyte solution and then applying a voltage.

The residual chlorine measurement apparatus 100 comprises, as basic components thereof, as shown in Figure 2-1, a working electrode 102, a reference electrode 103, a counter electrode 104, a potentiostat 105 for controlling the potential of the working electrode 102, the reference electrode 103 and the counter electrode 104, and an information processing device 106 for calculating the residual chlorine concentration of a sample solution, pH of the sample solution, and the like based on the current and potential obtained by the potentiostat 105 (also referred to as the first information processing device).

The sample solution 101 may be any sample solution as long as the same has the possibility of containing residual chlorine, which is the object being measured, and in the present embodiment, sodium hypochlorite (NaClO) is used. Further, as an electrolyte, a 0.1M sodium perchlorate ($NaClO_4$) is used. Hypochlorous acid can be present as hypochlorite ion and/or hypochlorous acid, or chlorine depending on the pH of the sample solution. Incidentally, although a reference sign is assigned to the sample solution 101 for convenience of description, this does not mean that the sample solution is a constituent of the apparatus 100. The apparatus 100 of the present invention can be used for any sample(s). Examples of the sample solution include, but are not limited to, phosphate buffer solutions (PBS), tap water, drinking water, river water, industrial wastewater, industrial waste liquids and test reagents.

The working electrode 102 is for applying a voltage to the sample solution, and in one embodiment, the same is a boron-doped conductive diamond electrode having conductivity due to boron doping.

Further, with the information processing device 106, the potential of the working electrode against the reference electrode can be swept in the range of from 0 V to +1.6 V, for example, in the range of from +0.1 V to +1.6 V, in the range of from +0.2 V to +1.6 V, in the range of from +0.3 V to +1.6 V, in the range of from +0.4 V to +1.6 V, in the range of from +0.5 V to +1.6 V, in the range of from +0.6 V to +1.6 V, in the range of from +0.7 V to +1.6 V, in the range of from +0.8 V to +1.6 V, in the range of from +0.9 V to +1.6 V, in the range of from +1.0 V to +1.6 V, in the range of from +1.1 V to +1.6 V, in the range of from +0 V to +1.5 V, in the range of from 0 V to +1.45 V, in the range of from 0 V to +1.4 V, or for example, in the range of from 0.4 V to +1.6 V; and when doing so, the sweep is carried out by starting from a low potential on the 0 V side and in the direction of a high potential on the +1.6 V side. Further, with the information processing device 106, the potential of the working electrode against the reference electrode can be swept in the range of from +0.4 V to -1.0 V, for example, in the range of from +0.4 V to -0.9 V, in the range of from +0.4 V to -0.8 V, in the range of from +0.3 V to -1.0 V, in the range of from +0.2 V to -1.0 V, in the range of from +0.1 V to -1.0 V, in the range of from 0 V to -1.0 V, in the range of from -0.1 V to -1.0 V, in the range of from -0.2 V to -1.0 V, in the range of from -0.3 V to -1.0 V or in the range of from -0.4 V to -1.0 V; and when doing so, the sweep is carried out by starting from a high potential on the +0.4 V side and in the direction of a low potential on the -1.0 V side.

A conductive diamond electrode is used for the working electrode 102 of the present invention. It is preferable that the conductive diamond electrode is doped with a minute amount of impurities. Being doped with impurities confers a desirable property as an electrode. The impurities include boron (B), sulfur (S), nitrogen (N), oxygen (O) and silicon (Si) and the like. For example, to a raw material gas containing a carbon source, in order to confer boron, diborane, trimethoxyborane or boron oxide can be added; in order to confer sulfur, sulfur oxide or hydrogen sulfide can be added; in order to confer oxygen, oxygen or carbon dioxide can be added; in order to confer nitrogen, ammonia or nitrogen can be added; and in order to confer silicon, silane or the like can be added. In particular, a conductive diamond electrode doped with boron at high concentrations is preferable since the same has advantageous properties of a broad potential window and a lower background current compared with other electrode materials. As such, in the present invention (disclosure), the boron-doped diamond electrode will be described illustratively below. Conductive diamond electrodes doped with other impurities may also be used. In the present specification, unless specified otherwise, the potential and the voltage are used as being synonymous and are mutually interchangeable. Further, in the present specification, the conductive diamond electrode may simply be described as a diamond electrode, and the boron-doped diamond electrode may simply be described as a BDD electrode.

An electrode unit (part) of the working electrode 102 of the present invention comprises a diamond layer made by vapor deposition of a diamond mixed with 0.01 to 8% w/w boron raw material on a substrate surface. The size of the substrate is not particularly limited. However, a substrate having an area capable of measuring a sample solution in milliliters or microliters is preferable. The substrate can be a substrate having, for example, a diameter of 1 to 10 cm and a thickness of 0.1 mm to 5 mm. The substrate can be a Si substrate, a glass substrate of $SiO_2$ and the like, a quartz substrate, a ceramic substrate of $Al_2O_3$ and the like, or a metal substrate of tungsten, molybdenum and the like. All or part of the surface of the substrate can be a diamond layer.

The size of the electrode unit of the conductive diamond electrode of the present invention can be suitably designed according to the object being measured. For example, the electrode unit may have a surface comprising an area of, for example, 0.1 $cm^2$ to 10 $cm^2$, 0.2 $cm^2$ to 5 $cm^2$, or 0.5 $cm^2$ to 4 $cm^2$. All or part of the diamond layer can be used for electrochemical measurement. Those skilled in the art can suitably determine the area and shape of the electrode unit depending on the object being measured.

The electrode unit of the working electrode 102 of the present invention comprises a diamond layer made by vapor-deposition of diamond mixed with high amounts of boron raw material (0.01 to 8% w/w boron raw material as the introduced raw material) on a Si substrate surface. The boron raw material mixing ratio is preferably 0.05 to 5% w/w, and particularly preferably about 1.0% w/w.

The vapor-deposition treatment of the diamond mixed with boron raw material onto the substrate may be carried out at 700 to 900°C for 2 to 12 hours. The conductive diamond thin film is produced by a typical microwave plasma chemical vapor deposition (MPCVD). That is, a substrate such as a silicon single crystal (100) is set in a film depositing apparatus, and a gas for film deposition using a high-purity hydrogen gas as a carrier gas is injected. The gas for film deposition contains carbon and boron. By radiating a microwave on the film depositing apparatus to which the high-purity hydrogen gas containing carbon and boron is injected, to cause plasma discharge, carbon radicals are generated from the carbon source in the gas for film deposition and are deposited on the Si single crystal while maintaining $sp^3$ structure and with boron being mixed, to thereby form a diamond thin film.

The film thickness of the diamond thin film can be controlled by adjusting the film formation time. The thickness of the diamond thin film can be, for example, 100 nm to 1 mm, 1 $\mu$m to 100 $\mu$m, 2 $\mu$m to 20 $\mu$m or the like.

The condition of the vapor-deposition treatment of boron-doped diamond on the substrate surface may be determined depending on the substrate material. As an example, the plasma output can be set to 500 to 7,000 W, for example, 3 kW to 5 kW, preferably 5 kW. When the plasma output is in this range, synthesis proceeds efficiently and a high quality diamond thin film with little by-products is formed.

In one embodiment, the above boron-doped conductive diamond electrode is preferably hydrogen-terminated or cathodically reduced. This is because, when compared with the case where the oxidation current and/or the reduction current is measured by using an oxygen-terminated or anodically oxidized boron-doped conductive diamond electrode, voltage values at which respective peak currents are detected become observable in the more inner side of the potential window, and sensitivity and precision are improved. Incidentally, the more inner side of the potential window refers to the side where the absolute value of the voltage value is lower. For example, when the oxidation current is measured under a certain condition, in the case of oxygen-terminated diamond, a peak current is observed at +2 V whereas in the case of hydrogen-terminated diamond, a peak current is observed at +1 V. Further, when the reduction current is measured under a certain condition, in the case of oxygen-terminated diamond, a peak current is observed at -2 V whereas in the case of hydrogen-terminated diamond, a peak current is observed at -1 V. Such cases are referred to as cases where the voltage values at which respective peak currents are detected are observed in the more inner side of the potential window.

Specific methods of hydrogen-termination include subjecting the conductive diamond electrode to hydrogen plasma treatment or annealing (heating) in a hydrogen atmosphere. Specific examples of a method of cathodic reduction include applying a potential of -3 V for 5 to 10 min in a 0.1M sodium perchlorate solution to continuously generate hydrogen.

Specific methods of oxygen-termination include subjecting the conductive diamond electrode to oxygen plasma treatment or annealing (heating) in an oxygen atmosphere (in the air). Specific examples of anodic oxidation include, e.g., applying a potential of +3 V for 5 to 10 min in a 0.1M sodium perchlorate solution to continuously generate oxygen.

The electrode above is disclosed in JP Patent Publication (Kokai) No. 2006-98281, JP Patent Publication (Kokai) No. 2007-139725, JP Patent Publication (Kokai) No. 2011-152324, JP Patent Publication (Kokai) No. 2015-172401 or the like, and can be produced according to the descriptions in these Publications.

The conductive diamond electrode of the present invention has high thermal conductivity, has high hardness, is chemically inert, has a broad potential window, has a low background current and is excellent in electrochemical stability.

A production example of the electrode will be shown. In one embodiment, the conductive diamond electrode was produced by a chemical vapor deposition (CVD) method. The apparatus used was an AX5400, manufactured by Cornes Technologies Ltd. Acetone was used as the carbon source, and $B(OCH_3)_3$ was used as the boron source. The concentration accounted for by $B(OCH_3)_3$ in the raw material was 8.7% w/w (in the case of a boron concentration of 1%). The (100) surface of a silicon substrate was nucleated with a diamond powder, and a film was formed on the substrate under the condition of a plasma output of 5,000 W for about 6 hours with a pressure of 110 Torr. The area of the working electrode was made to be 1 $cm^2$.

In the first embodiment, the apparatus 100 of the present invention comprises the three electrodes. The resistance of the reference electrode 103 side is set at a high resistance, and no current flows between the working electrode 102 and the reference electrode 103. The counter electrode 104 is not particularly limited to any material and, for example, silver wire, platinum wire, carbon, stainless steel, gold, diamond, $SnO_2$ or the like can be used. Examples of the reference electrode 103 include a silver/silver chloride electrode (Ag/AgCl), a standard hydrogen electrode, a mercury/mercury chloride electrode, a hydrogen palladium electrode and the like, but is not limited thereto. In one embodiment, the reference electrode 103 can be a silver/silver chloride electrode (Ag/AgCl) from the perspective of stability, reproducibility and the like. In the present specification, unless otherwise specified, measured voltages are those measured with reference to a silver/silver chloride electrode (+0.199 V vs. a standard hydrogen electrode (SHE)). The shapes, the sizes and the positional relations of the working electrode 102, the reference electrode 103 and the counter electrode 104 can suitably be designed, but each of the working electrode 102, the reference electrode 103 and the counter electrode 104 are designed and arranged so as to be simultaneously contactable with the sample being measured.

The silver/silver chloride electrode to be used as the reference electrode 103 is composed of a AgCl-coated silver wire (Ag/AgCl) immersed in an aqueous solution containing chloride ion ($Cl^-$). The counter electrode 104 is not particularly limited as long as the same has a larger surface area than that of the working electrode 102.

The potentiostat 105 comprises a voltage applying section to apply voltages to the working electrode 102, the reference electrode 103 and the counter electrode 104, as well as a current measuring section to measure current values under the applied voltages. The potentiostat 105 receives voltage signals and current signals from the working electrode 102, the reference electrode 103 and the counter electrode 104, and, along with this, controls the working electrode 102, the reference electrode 103 and the counter electrode 104. More specifically, the potentiostat 105 adjusts the voltage applied between the working electrode 102 and the counter electrode 104 at all times, and controls voltages of the working electrode 102 against the reference electrode 103. The potentiostat 105 is controlled by the information processing device 106.

In one embodiment, the potentiostat 105 scans the potential of the working electrode 102 against the reference electrode 103 from 0 V to +1.6 V, for example, at a rate of 100 mV/sec, and detects current values accompanied by the oxidation reaction under the voltages.

Further, in one embodiment, the potentiostat 105 scans the potential of the working electrode 102 against the reference electrode 103 from +0.4 V to -1.0 V, for example, at a rate of 100 mV/sec, and detects current values accompanied by the reduction reaction under the voltages.

The information processing device 106 comprised by the apparatus 100 of the present invention controls the potentiostat 105, determines a current-voltage curve based on voltage signals and current signals from the potentiostat 105, and calculates the residual chlorine concentration in a sample solution based on the current-voltage curve.

In one embodiment, the information processing device 106 comprised by the apparatus of the present invention controls the potentiostat 105 so that the potential of the working electrode 102 against the reference electrode 103 is altered from 0 V to +1.6 V, for example, at a rate of 100 mV/sec. In one embodiment, the information processing device 106 comprised by the apparatus 100 of the present invention controls the potentiostat 105 so that the potential of the working electrode 102 against the reference electrode 103 is altered from +0.4 V to -1.0 V, for example, at a rate of 100 mV/sec.

In one embodiment, the apparatus 100 of the present invention has a second information processing device 140. In such case, for the sake of convenience, the information processing device of 106 may also be referred to as a first information processing device 106. In one embodiment, the second information processing device 140 is connected to the temperature measuring unit 120 and the pH measuring unit 130, and controls the temperature measuring unit 120 and/or the pH measuring unit 130, and receives and processes measurement results. In another embodiment, the second information processing device 140 is connected to a temperature measuring unit 220 and a pH measuring unit 230, and controls the temperature measuring unit 220 and/or the pH measuring unit 230, and receives and processes measurement results.

The first information processing device 106 and/or the second information processing device 140 comprised by the apparatus 100 of the present invention may comprise a CPU, an internal memory, an external memory medium or device such as a HDD, a communication interface such as a modem or a wireless LAN, a display, input means such as a mouse and a keyboard, and the like. The first information processing device 106 and/or the second information processing device 140 can analyze electric signals according to a program set in a predetermined region of the memory, the external memory device or the like, and carry out the detection of the residual chlorine and the calculation of the concentration. The first information processing device 106 and/or the second information processing device 140 may be a general computer or may be a dedicated computer. The second information processing device 140 may be connected to the first information processing device 106. The information processing device 106 may play the roles of the first information processing device and the second information processing device.

Results of voltammetric measurement of sample solutions using the residual chlorine measurement apparatus 100 according to the present embodiment are shown in Figure 3, Figure 4 and Figure 5. Figure 5 is a figure in which Figure 3 and Figure 4 are connected.

The sample solutions 101 are eight 100-ppm NaClO solutions adjusted at a pH of 2 to 9 by using $HClO_4$ and NaOH.

Figure 3 shows voltammograms of the eight sample solutions having different pHs measured by sweeping the potential of the working electrode 102 against the reference electrode 103 from +0.4 V to +2.0 V (at a rate of 20 mV/sec). Despite the same residual chlorine concentration (100-ppm NaClO), different oxidation current values (hypochlorite ion concentrations) were obtained depending on pH. That is, at a pH of 9, since about 97% of the residual chlorine exists as hypochlorite ion as seen in Figure 1, large oxidation currents are measured; and at a pH of 5, since nearly 100% of the residual chlorine is hypochlorous acid and hypochlorite ion does not exist, almost no oxidation currents are measured. As the pH of the sample solutions decreases from 9 toward 5, the compositional ratio of hypochlorite ion becomes low as seen in Figure 1 and, therefore, the oxidation current also decreases as the pH decreases.

Figure 4 shows voltammograms of the eight sample solutions having different pHs measured by sweeping the potential of the working electrode 102 against the reference electrode 103 from +0.4 V to -1.6 V (at a rate of 20 mV/sec). Despite the same residual chlorine concentration (100-ppm NaClO), different reduction current values (hypochlorous acid concentrations) were obtained depending on pH. That is, at a pH of 9, since only about 3% of the residual chlorine exists as hypochlorous acid as seen in Figure 1, almost no reduction currents are measured; while at a pH of 5, since nearly 100% of the residual chlorine exists as hypochlorous acid, large reduction currents are measured. As the pH of the sample solutions decreases from 9 toward 5, the compositional ratio of hypochlorous acid becomes high as seen in Figure 1 and, therefore, the reduction current increases as the pH decreases.

Figure 5 shows voltammograms produced by connecting the results indicated in Figure 3 and Figure 4.

In a sample solution having any pH of 5 or higher, by adding the concentration of hypochlorite ion determined by using a calibration curve for determining the hypochlorite ion concentration from the oxidation current (hereinafter, referred to as oxidation-side calibration curve. The production method of the same will be described later.), and the concentration of hypochlorous acid determined by using a calibration curve for determining the concentration of hypochlorous acid from the reduction current (hereinafter, referred to as reduction-side calibration curve. The production method of the same will be described later.), the residual chlorine concentration of the sample solution having any pH can be determined.

Figure 6 shows the residual chlorine concentration measured by the method mentioned above. For the sample solution 101, eight 100-ppm NaClO solutions adjusted to a pH of 2 to 9 by using $HClO_4$ and NaOH as described above were used. The figure shows concentrations obtained by adding the concentration of hypochlorite ion and the concentration of hypochlorous acid, determined from the oxidation-side calibration curve and the reduction-side calibration curve. When the hypochlorite ion concentration measurement and the hypochlorous acid concentration measurement are carried out continuously by using the same sample solution 101, it is necessary to carry out the measurements while stirring the sample solution 101. Stirring may be carried out by external means. Alternatively, stirring means may be attached to the apparatus 100. The hypochlorite ion concentration measurement and the hypochlorous acid concentration measurement may also be carried out by using sample solutions 101 different from each other.

According to Figure 6, in the range of a pH of 4 to 9, it was shown that 100 ppm residual chlorine concentration of the sample solution 101 could be measured by adding the concentration of hypochlorite ion and the concentration of hypochlorous acid. At a pH of 4, the compositional ratio (hereinafter, also referred to as abundance ratio) of dissolved chlorine is small and this does not appear as an error in the reduction current and, therefore, it is believed that the residual chlorine concentration is being measured accurately.

Production of the calibration curves

The calibration curves can be produced as follows.

1. The oxidation-side calibration curve

(1) Sample solutions 101 having a pH of for example 9 are prepared for each concentration of residual chlorine (for example, 0 ppm, 20 ppm, 40 ppm, 60 ppm, 80 ppm, 100 ppm and the like). The concentration of hypochlorite ion in the case of a pH of 9 is 97% of the residual chlorine concentration and, therefore, the hypochlorite ion concentration of the sample solutions 101 in the case of a pH of 9 becomes, for example, 0 ppm, 19.4 ppm, 38.8 ppm, 58.2 ppm, 77.6 ppm, 97 ppm and the like. Next, the oxidation currents of the sample solutions 101 are measured. An example of measurement results is shown in Figure 7. As seen in Figure 7, large response currents (oxidation currents) were observed as the residual chlorine concentration increased. Based on this result, a graph of the hypochlorite ion concentration vs. the oxidation current is produced to thereby generate the oxidation-side calibration curve. Figure 8 shows an example of the oxidation-side calibration curve produced based on the measurement results of Figure 7.

(2) Sample solutions 101 having a residual chlorine concentration of, for example, 100 ppm and a pH of 4 or higher, for example, various different pHs of 5 or higher are prepared. Among the various sample solutions 101 prepared, the oxidation current of a first sample solution 101 is measured. When the pH of the first sample solution 101 is, for example, 7, the hypochlorite ion compositional ratio is 23% as recognized from Figure 1. As such, it is judged that the measured oxidation current corresponds to the case where the hypochlorite ion concentration is 23 ppm, and this is plotted on a graph of the hypochlorite ion concentration vs. the oxidation current. Next, the oxidation current of a second sample solution 101 is measured. When the pH of the second sample solution 101 is, for example, 8, the hypochlorite ion compositional ratio is 75% as recognized from Figure 1. As such, it is judged that the measured oxidation current corresponds to the case where the hypochlorite ion concentration is 75 ppm, and this is plotted on the graph of the hypochlorite ion concentration vs. the oxidation current. This procedure is repeated to carry out the measurement of the prepared sample solutions 101 having various pHs to thereby generate the oxidation-side calibration curve.

(3) The oxidation-side calibration curve can also be produced by combining (1) and (2) above.

2. The reduction-side calibration curve

(1) Sample solutions 101 having a pH of for example, 6 are prepared for each concentration of residual chlorine (for example, 0 ppm, 20 ppm, 40 ppm, 60 ppm, 80 ppm, 100 ppm and the like). The concentration of hypochlorous acid in the case of a pH of 6 is 97% of the residual chlorine concentration and, therefore, the concentration of hypochlorous acid of the sample solutions 101 in the case of a pH of 6 becomes, for example, 0 ppm, 19.4 ppm, 38.8 ppm, 58.2 ppm, 77.6 ppm, 97 ppm and the like. Next, the reduction currents of the sample solutions 101 are measured. An example of measurement results is shown in Figure 9. As seen in Figure 9, large response currents (reduction currents) were observed as the residual chlorine concentration increased. Based on this result, a graph of the hypochlorous acid concentration vs. the reduction current is produced to thereby generate the reduction-side calibration curve. Figure 10 shows an example of the reduction-side calibration curve produced based on the measurement results of Figure 9.

(2) Sample solutions 101 having a residual chlorine concentration of, for example, 100 ppm and a pH of 4 or higher, for example, various pHs of 5 or higher are prepared. The reduction current of a first sample solution 101 among the prepared various sample solutions 101 is measured. When the pH of the first sample solution is, for example, 7, the hypochlorous acid compositional ratio is 77% as recognized from Figure 1. As such, it is judged that the measured reduction current corresponds to the case where the concentration of hypochlorous acid is 77 ppm, and this is plotted on a graph of the hypochlorous acid concentration vs. the reduction current. Then, the reduction current of a second sample solution 101 is measured. When the pH of the second sample solution 101 is, for example, 8, the hypochlorous acid compositional ratio is 25% as recognized from Figure 1. As such, it is judged that the measured reduction current corresponds to the case where the concentration of hypochlorous acid is 25 ppm, and this is plotted on the graph of the hypochlorous acid concentration vs. the reduction current. This procedure is repeated to carry out the measurement of the prepared sample solutions 101 having the various pHs to thereby generate the reduction-side calibration curve.

(3) The reduction-side calibration curve can also be produced by combining (1) and (2) above.

By using the calibration curves produced in advance, the response currents can be measured for a sample solution with unknown residual chlorine concentration, and the residual chlorine concentration can be determined from the measured response currents.

<Second embodiment>

Below, a second embodiment of the residual chlorine concentration measuring apparatus according to the present invention will be described. The same reference signs will be used for elements corresponding to those in the first embodiment.

The residual chlorine measurement apparatus 200 according to the second embodiment of the present invention comprises a working electrode 102, a reference electrode 103, a counter electrode 104, a potentiostat 105 and an information processing device 106 similar to those in the first embodiment; however, these elements have shapes and arrangements as shown in Figure 11-1.

The residual chlorine measurement apparatus 200 according to the second embodiment of the present invention is for carrying out flow injection analysis (FIA). Flow injection analysis (FIA) is a method in which a sample is injected in a flowing solution and components in the solution are analyzed in a flow cell through which the flowing solution passes. In general, a flow injection analysis system comprises means of generating flowing such as a metering pump, and comprises a detector comprising a flow cell. Controlled continuous flowing is generated by the metering pump or the like. Various reactions, separation, sample injection and the like can be carried out in this flow (flowing). Further, components in the solution can be analyzed by the detector comprising a flow cell.

An example of the flow injection analysis system is shown in Figure 11-1. As shown in Figure 11-1, the apparatus 200 comprises a flow tube 211 through which a sample solution 101 passes, a pump 209 for passing the sample solution 101 through the flow tube 211, and a flow cell 207 through the interior of which the flow tube 211 passes. The flow cell 207 comprises the working electrode 102, reference electrode 103 and counter electrode 104 built-in. The working electrode 102, the reference electrode 103 and the counter electrode 104 are connected to the potentiostat 105 through wirings 110. Further, the potentiostat 105 is connected to the information processing device 106. The flow tube 211 comprises an inflow port 213 to the flow cell 207 and an outflow port 214 from the flow cell 207. The interior of the flow tube 211 is referred to as a flow path 212.

The sample solution 101 is a sample solution possibly containing (having the possibility of containing) residual chlorine, the object being measured; and in the present embodiment, sodium hypochlorite (NaClO) is used. Further, as an electrolyte, a 0.1M sodium perchlorate ($NaClO_4$) is used.

A channel through which the sample solution 101 passes is constituted of the flow tube 211 and the flow cell 207. The flow tube 211 connects a solution tank 208 with the inflow port 213 of the flow cell 207. Though not shown in figure, the outflow port 214 of the flow cell 207 can be connected to a waste liquid tank. The pump 209 is arranged preferably not on the outflow port 214 side of the flow cell 207 but on the inflow port 213 side (upstream side). The pump 209 can feed the sample solution 101 to the flow cell 207 at a constant rate. Examples of the pump include a pump for liquid chromatography and the like.

The working electrode 102, the reference electrode 103 and the counter electrode 104 built in the flow cell 207 are exposed in the flow path 212 so as to be able to contact with the sample solution 101. In particular, the diamond thin film of the working electrode 102 is exposed in the flow path 212, and when the sample solution 101 passes, this can contact with the sample solution 101. The sample solution 101 goes from the inflow port 213 into the flow cell 207, flows in the direction of the arrow in the figure, and is fed to the outflow port 214. When the sample solution 101 is contacted with the electrodes, electrochemical reaction occurs in the sample solution 101 when a voltage is applied between the working electrode 102 and the reference electrode 103.

Next, the operation of the residual chlorine measurement apparatus 200 is described.

The sample solution 101 possibly containing residual chlorine, the object being measured, is fed by the pump 209 from the solution tank 208 through the flow tube 211 to the flow cell 207. In the flow cell 207, under conditions where the built-in working electrode 102, reference electrode 103 and counter electrode 104 are in contact with the sample solution 101, electrochemical reaction occurs by applying voltages between the working electrode 102 and the reference electrode 103. Current values (electric signals) produced by the electrochemical reaction are transmitted to the potentiostat 105 and the control and detection of signals at each of the electrodes are carried out. The signals detected by the potentiostat 105 are analyzed by the information processing device 106, and detection of residual chlorine and measurement of the residual chlorine concentration are carried out. The sample solution 101 after measurement is finished is discharged through the outflow port 214 outside the flow cell 207.

In the present embodiment, residual chlorine concentration can be measured as follows.

(1) A continuous potential sweeping system

(i) The potential to be applied to the working electrode 102 is swept repeatedly between -1.6 V and +2.0 V. For example, the potential is swept from +0.4 V to +2.0 V, then, from +0.4 V to -1.6 V, and again from +0.4 V to +2.0 V, then, +0.4 V to -1.6 V. Then, this is repeated for an arbitrary number of times.
(ii) The oxidation current is measured when the potential applied to the working electrode becomes +1.4 V, and the oxidation-side residual chlorine concentration is calculated by using the calibration curve produced in advance.
(iii) Further, the reduction current is measured when the potential applied to the working electrode becomes -0.5 V, and the reduction-side residual chlorine concentration is calculated by using the calibration curve produced in advance.
(iv) The concentration obtained by adding the oxidation-side residual chlorine concentration and the reduction-side residual chlorine concentration is designated as (is regarded as) the residual chlorine concentration of the sample solution.
(v) Optionally, from the ratio of the oxidation-side residual chlorine concentration and the reduction-side residual chlorine concentration obtained in step (iv), the pH of the sample solution can also be calculated.

(2) A potential switching system

(i) The potential to be applied to the working electrode 102 is switched alternately between -0.5 V and +1.4 V. For example, the potential is held at -0.5 V for a certain time, then held at +1.4 V for a certain time, then again held at -0.5 V for a certain time, and then again held at +1.4 V for a certain time. This is repeated for an arbitrary number of times.
(ii) The oxidation current is measured when the potential applied to the working electrode is held at +1.4 V, and the oxidation-side residual chlorine concentration is calculated by using the calibration curve produced in advance.
(iii) The reduction current is measured when the potential applied to the working electrode is held at -0.5 V, and the reduction-side residual chlorine concentration is calculated by using the calibration curve produced in advance.
(iv) The concentration obtained by adding the oxidation-side residual chlorine concentration and the reduction-side residual chlorine concentration is designated as the residual chlorine concentration of the sample solution.

(v) Optionally, from the ratio of the oxidation-side residual chlorine concentration and the reduction-side residual chlorine concentration obtained in step (iv), the pH of the sample solution can also be calculated.

(3) A six-electrode system

The three electrodes of the working electrode, the counter electrode and the reference electrode are taken as one set, and two sets thereof, i.e., six electrodes, are incorporated in the flow cell.

(i) The potential to be applied to the working electrode of one set is fixed at +1.4 V. Further, the potential to be applied to the working electrode of the other set is fixed at -0.5 V.
(ii) The oxidation current of the side in which the potential applied to the working electrode is fixed at +1.4 V is measured, and the oxidation-side residual chlorine concentration is calculated by using the calibration curve produced in advance.
(iii) The reduction current of the side in which the potential applied to the working electrode is fixed at -0.5 V is measured, and the reduction-side residual chlorine concentration is calculated by using the calibration curve produced in advance.
(iv) The concentration obtained by adding the oxidation-side residual chlorine concentration and the reduction-side residual chlorine concentration is designated as the residual chlorine concentration of the sample solution.
(v) Optionally, from the ratio of the oxidation-side residual chlorine concentration and the reduction-side residual chlorine concentration obtained in step (iv), the pH of the sample solution can also be calculated.

(4) A four-electrode system

Two working electrodes, one counter electrode and one reference electrode are incorporated in the flow cell. As the potentiostat, a bipotentiostat is used.
The bipotentiostat (also referred to as dual potentiostat) is a potentiostat capable of controlling two working electrodes, and can individually control the two working electrodes inserted in one solution system and measure the respective response currents. In order to use a bipotentiostat, in general, one counter electrode and one reference electrode will suffice. In the bipotentiostat, a circuit to control the potential of the other working electrode is added to a usual potentiostat. As the bipotentiostat of the present invention, any known bipotentiostat can be used. For example, see Bard, et al., L.R., Electrochemical Methods: Fundamentals and Applications, New York: John Wiley & Sons, 2nd Edition, 2000; Handbook of Electrochemistry, Elsevier, 2007; Kissinger et al. Laboratory Techniques in Electroanalytical Chemistry, CRC Press, 1996; and the like.
In the case of carrying out the measurement by using the four-electrode system, the operation is carried out basically in the similar manner as in the case of the above (3) six-electrode system.
In one embodiment, the method and the apparatus of the present invention can accurately measure the residual chlorine concentration by making the temperature correction even when the temperature of the solution has varied. In another embodiment, the method and the apparatus of the present invention can determine the pH of the solution by making the temperature correction even when the temperature of the solution has varied. In another embodiment, the apparatus of the present invention may calculate whether or not the measurement result has a value within a predetermined error and carry out self-diagnosis of the measuring instrument.

<Third embodiment>

Below, a third embodiment of the residual chlorine concentration measuring apparatus according to the present invention will be described.
In one embodiment, the apparatus of the present invention further comprises a temperature measuring unit of the solution. In one embodiment, a residual chlorine concentration measuring method is provided in which a temperature correction value is calculated from the solution temperature measured with said temperature measuring unit, and a residual chlorine concentration obtained by making a temperature correction using the temperature correction value in the residual chlorine concentration calculated by the residual chlorine concentration measuring method of the present invention is designated as the residual chlorine concentration of the solution. The temperature measuring unit may be incorporated integrally in the apparatus, or may be installed separately as an independent unit.
The temperature measuring unit comprises any conventional temperature measuring means, for example, a conventional thermometer. As the temperature measuring means, any temperature measuring means can be used as long as the means can measure the temperature of the solution. Examples of the temperature measuring means include thermocouples, resistance thermometers, liquid column thermometers, glass thermometers, semiconductors and the like, but are not limited thereto. For the temperature measuring means, a temperature measuring means having a lower heat capacity is preferable. For example, when the solution cooled down to 4°C is made to flow in the flow cell placed at room temperature and when the changes of the temperature of the solution flowing in the cell is measured by a platinum temperature sensor or a thermocouple, when the heat capacity of the temperature sensor is large, the time until temperature equilibrium is reached is prolonged (see Figure 23). Therefore, in one embodiment, the temperature measuring unit comprises a temperature measuring means having a small heat capacity, for example, a thermocouple having a small heat capacity.

In Figure 2-2, an apparatus comprising a temperature measuring unit 120 is shown as a modified method of the first embodiment. The temperature measuring unit 120 is connected to a second information processing device 140. Further as a modified method of the second embodiment, an apparatus comprising a temperature measuring unit 220 is shown in Figure 11-2. The temperature measuring unit 220 is connected to a second information processing device 140.

In one embodiment, a pH measuring method is provided in which, with regard to the pH calculated by the pH measuring method of the present invention, the abundance ratio of hypochlorous acid and hypochlorite ion is calculated from the solution temperature measured with a temperature measuring unit, and the temperature corrected pH of the solution is designated as the pH of the solution.

< 1. A preliminary test-temperature dependency of the chlorine concentration measurement by a spectrophotometer>

Briefly, the chlorine concentration measurement using a spectrophotometer was carried out on the same solution at various temperatures.

The spectrophotometer was AQ-202, manufactured by Shibata Scientific Technology Ltd.; and the measurement solution was chlorinated water produced by a Well Clean TE, manufactured by OSG Corp. When the temperature of the measuring solution was altered and the chlorine concentration was measured by the spectrophotometer, differences as large as 21 ppm occurred in the range of the solution temperature of 6°C to 38°C. Results are shown in Figure 12. From this result, it can be recognized that measurement of correct chlorine concentrations for various solution temperatures is difficult using a spectrophotometer.

As such, the solution temperature of 25°C was defined as the standard state; a value by a spectrophotometer at the standard state was designated to be the true value; and based on the premise that chlorine concentration (ppm) does not change even when the solution temperature changes, a method for estimating the chlorine concentration for the solution having a different temperature was established.

<2. Determination of temperature coefficient (chlorine concentration)>

Determination of the temperature coefficient was carried out with the following procedure.

(i) First, chlorinated water solutions having different concentrations are generated. Chlorinated water produced by the chlorinated water producing apparatus was used as an undiluted solution, and this was diluted successively with a NaCl solution.

(ii) Next, the solution temperature was adjusted to 25°C, and the respective residual chlorine concentrations of the solutions were measured.

(iii) Next, the solution temperature was altered and the pH at the respective temperature was measured. The pH was measured by pH meter LAQUA twin, manufactured by HORIBA, Ltd. The residual chlorine concentration was separated to concentrations of [HClO] and [$ClO^-$] based on the pH after the temperature variation (Figure 21-1).

$$\text{Residual chlorine concentration (ppm)} = [\text{HClO}](\text{ppm}) + [\text{ClO}^-](\text{ppm})$$

(iv) Next, a flow cell was assembled. The electrode unit of the flow cell comprised a construction such that the working electrode was a conductive diamond electrode; the reference electrode was a silver/silver chloride electrode; and the counter electrode was a conductive diamond electrode. The oxidation current and the reduction current of the solution were measured using the assembled flow cell. The experimental was carried out with the sample solution flowing in the flow cell and the experiment condition was a flow rate of about 120 mm/sec. The current measurement was carried out at a constant potential, and the potential to be used in the measurement and the time at which the current value was to be read were determined in advance. Results are shown in Figures 14 and 15.

(v) The relation between the observed oxidation current and [$ClO^-$](ppm) and the relation between the observed reduction current and [HClO](ppm) were graphed, and regression lines were determined. The gradients (slopes) of the regression lines indicate current sensitivities at the respective concentrations.

(vi) The series of steps (iii) to (v) above were carried out for a plurality of solution temperatures, and current sensitivities at the respective solution temperatures were determined (Figure 16).

(vii) The ratios of the gradients at the respective solution temperatures determined in step (vi) above were calculated. Results are shown in Table 1.

[Table 1]

| Solution Temperature | Gradient | Ratio of Gradients (Ratio of Current Values) |
|---|---|---|
| 7.8 | -2.53 | 0.54 |
| 13.5 | -3.14 | 0.67 |
| 20.8 | -4.20 | 0.90 |
| 25.0 | -4.67 | 1.00 |
| 28.8 | -5.09 | 1.09 |
| 35.5 | -6.13 | 1.31 |

The gradient in the case of 25°C was -4.67. This was designated as the basis (1.0) and the ratio of the gradients at each temperature was calculated. The "ratio" of the gradients serves as the "temperature coefficient" of the current sensitivity for the solution temperature. That is, in the present specification, the temperature coefficient is defined as the ratio of a current value observed at a specific temperature to a current value observed at the standard temperature determined by the series of steps above. Although 25°C was set as the standard temperature in the above example for the sake of convenience, the standard temperature can be set at any given temperature.

<3. Correction based on the temperature coefficient (chlorine concentration)>

By using the temperature coefficient determined as described above, correction based on the temperature coefficient is carried out by the following procedure. The flow cell used was the same as in the above. The experimental condition is the same as that in step (iv) of

<2. Determination of temperature coefficient (chlorine concentration)>.

(i) When the oxidation current and the reduction current are measured by the flow cell, the temperature of the solution in the flow cell is measured. The temperature was measured by an in-house thermocouple.

(ii) The measured oxidation current and reduction current are converted to values at 25°C of the solution temperature by using the temperature coefficient determined in <2. Determination of temperature coefficient (chlorine concentration)> above.

For example, when the measured solution temperature is 15°C and the measured reduction current value is 200 μA, the temperature coefficient determined in <2. Determination of temperature coefficient (chlorine concentration) > above is +0.0277, and this shows that as the solution temperature increases by 1°C, the current value increases by 0.0277 fold. When the measured solution temperature is 15°C, the difference with the standard temperature of 25°C is 10°C. As such, the current value is calculated as increasing by 0.277 fold. The observed current value of 200 μA is, when the solution temperature is 25°C, estimated to be 200 × (1 + 0.277) = 255.4 μA (see Figure 17). When this value of 255.4 μA is plugged in to the straight line of 25°C in Figure 16, the concentration is 60 ppm (see Figure 18). The [HClO] concentration is determined to be 60 ppm from the measured solution temperature and reduction current value. The [HClO] concentration when no temperature correction is carried out is about 47 ppm, and the importance of temperature correction can be recognized.

<4. Determination of temperature coefficient (solution pH)>

The determination of the temperature coefficient was carried out by the following procedure.

(i) A calculation expression indicating the compositional ratio of hypochlorous acid and hypochlorite ion was determined by referencing the following paper:

"The Acid Ionization Constant of HOCl from 5 to 35°" by J. Carrell Morris, Division of Engineering and Applied Physics, Harvard University, Cambridge, Massachusetts (Received April 11, 1966)"

[Mathematical formula 1]

Proportion of $ClO^-$

$$\frac{[ClO^-]}{[HClO]+[ClO^-]}=\frac{1}{1+10^{(3000.00/T-10.0686+0.0253T-pH)}}$$

[Mathematical formula 2]

Proportion of HClO

$$\frac{[HClO]}{[HClO]+[ClO^-]}=\frac{1}{1+10^{(pH-3000.00/T+10.0686-0.0253T)}}$$

(ii) Results (temperature coefficients) of respective compositional ratios calculated by using the two expressions above are shown in Figure 21-1. Further, the temperature coefficients in the range of a pH of 7.3 to 8.0 are shown as an enlarged diagram in Figure 21-2.

<5. Correction based on the temperature coefficient (solution pH)>

By using the temperature coefficient determined as described above, correction based on the temperature coefficient is made by the following procedure.

(i) When the oxidation current and the reduction current are being measured by the flow cell, the temperature of the solution in the flow cell is measured.
(ii) When the reduction current only was observed:
It can be specified, from the compositional ratio curves of effective chlorine in Figure 21-1, that the pH of the sample solution is 5 or lower.
(iii) When the reduction current and the oxidation current were observed:
When the reduction current and the oxidation current are observed, the pH can be specified to be 5 < pH < 10, and the pH of the sample solution can be calculated from the compositional ratio curves of effective chlorine in Figure 21-1 by using the compositional ratio of the reduction-side residual chlorine concentration (residual chlorine concentration based on hypochlorite ion) and the oxidation-side residual chlorine concentration (residual chlorine concentration based on hypochlorous acid). For example, when the solution temperature is 25°C, and the reduction-side residual chlorine concentration = the oxidation-side residual chlorine concentration, then it can be understood that pH = 7.537. For example, when the solution temperature is 5°C, and the reduction-side residual chlorine concentration = the oxidation-side residual chlorine concentration, then it can be understood that pH = 7.754.
(iv) When the oxidation current only was observed:

It can be specified, from the compositional ratio curves of effective chlorine in Figure 21-1, that the pH of the sample solution is 10 or higher.
As above, the resultant pH is different for the case of 25°C and the case of 5°C, and the importance of the temperature correction can be recognized.

<Fourth embodiment>

Below, a fourth embodiment of the pH measuring apparatus according to the present invention is described.
In one embodiment, the apparatus of the present invention further comprises a pH measuring unit of the solution.

In one embodiment, the pH of the solution calculated by the measuring method of the present invention is compared with the pH of the solution measured by the pH measuring unit. When the difference between the pH of the solution calculated by the measuring method of the present invention and the pH of the solution measured by the pH measuring unit is within a predetermined error, then the apparatus can carry out self-diagnosis (self-inspection) that the measuring instrument is functioning normally. When the difference between the pH of the solution calculated by the measuring method of the present invention and the pH of the solution measured by the pH measuring unit exceeds the predetermined error, the apparatus can produce a signal indicating that the measuring instrument has an abnormality. The pH measuring unit may be incorporated integrally in the apparatus, or the pH measuring unit can be installed separately as an independent unit.

The pH measuring unit comprises a conventional pH measuring means, for example, a conventional pH meter. The pH measuring means may be any means so long as the same is capable of measuring the pH of a solution. Examples of the pH measuring means include pH meters utilizing a glass electrode method, and pH meters utilizing a solid phase electrode, but are not limited thereto. A pH measuring means having a lower heat capacity is preferable.

An apparatus comprising a pH measuring unit 130 is shown as a modified method of the first embodiment, in Figure 2-2. The pH measuring unit 130 is connected to a second information processing device 140. Further as a modified method of the second embodiment, an apparatus comprising a pH measuring unit 230 is shown in Figure 11-2. The pH measuring unit 230 is connected to a second information processing device 140.

In one embodiment, the measuring method of the present invention can be carried out at a constant potential (CA). In one embodiment, the apparatus of the present invention can carry out constant-potential measurement. The potential at which the constant-potential measurement is carried out, in the case of the oxidation current measurement, can be a given potential in the range of from 0 V to +2 V, for example, +0.1 V, +0.2 V, +0.5 V or +1.0 V, or for example, +2.0 V, but is not limited thereto. The potential, in the case of the reduction current measurement, can be a given potential in the range of from 0 V to -2 V, for example, -0.1 V, -0.2 V, -0.5 V or -1.0 V, or for example, -2.0 V, but is not limited thereto. The time during which the constant potential is applied can be about 1, about 2, about 3, about 4, about 5, about 6, about 7, about 8, about 9, about 10, about 15, about 20, about 30, about 40, about 50 or about 60 seconds, but is not limited thereto.

In one embodiment, the measuring method of the present invention further comprises an electrode initializing step (step of initializing the electrode). The electrode initialization can clean the electrode surface and recover the electrode performance.

The electrode initializing step may comprise
repeating the following steps (i) and (ii) as a pair one or more times (once or more):

(i) applying a positive or negative first pulse voltage for 0.01 to 60 sec; and
(ii) applying a negative or positive second pulse voltage, said second pulse voltage having a sign reverse to the pulse voltage applied in step (i), for 0.01 to 60 sec.

For example, steps (i) and (ii) are treated as a pair, and the pair may be repeated once, twice, three times ··· or n times. After the electrode initializing step, the reduction current measurement or the oxidation current measurement can appropriately be carried out. After the measurement, the electrode initializing step may again be carried out or not carried out, and the next measurement can be carried out. One example of electrode initialization pulses is shown in Figure 19. The pulse shape is not limited thereto. Further, the pulse voltage to be applied can be ±1 to 10 V, for example, ±1 to 10 V, ±1 to 9 V, ±1 to 8 V, ±1 to 7 V, ±1 to 6 V, ±1 to 5 V or ±1 to 4 V, or for example, ±1 to 3 V, or for example, ±1 to 2 V, but is not limited thereto. The time duration with which the pulse voltage is applied can be about 0.01, about 0.05, about 0.1, about 0.2, about 0.3, about 0.4, about 0.5, about 0.6, about 0.7, about 0.8, about 0.9, about 1, about 2, about 3, about 4, about 5, about 6, about 7, about 8, about 9, about 10, about 15, about 20, about 30, about 40, about 45, about 50 or about 60 sec, but is not limited thereto.

The effect of the electrode initializing step is shown in Figure 22. This indicates changes in results of current measurement when a linear sweep voltammmetry (LSV) measurement is repeated 300 times. Although initially a current peak indicating the presence of hypochlorous acid is observed in the vicinity of -0.7 V, as the LSV measurement is repeated, it becomes difficult to measure the current peak. Without wishing to be bound by any specific mechanism, it is presumed that the cause of decrease in measured current peak is fouling (deposits) on the working electrode surface, and the above shows that by carrying out an electrode initializing step, the sensitivity of the electrode returns to the initial property and it becomes possible to carry out measurement always in the same conditions.

In one embodiment, the measuring method of the present invention may comprise repeating the following steps (a) and (b) as a pair one or more times:

(a) carry out the electrode initializing step before the measurement; and
(b) then carrying out the constant-potential measurement.

For example, steps (a) and (b) are treated as a pair, and the pair may be repeated once, twice, three times ··· or n times. Further, an algorism that carries out such operation may be implemented in the apparatus of the present invention, the apparatus of the present invention may be controlled by a software that can execute such algorism, or such software may be stored in the apparatus of the present invention.

In one embodiment, the apparatus of the present invention comprises a silver electrode in place of a silver/silver chloride electrode as the reference electrode. In the case of using a silver electrode as the reference electrode, the silver electrode surface is, in general, treated with hydrochloric acid or the like to form a silver chloride film, and thereafter, the silver electrode is used as the reference electrode. However, the apparatus of the present invention in this embodiment comprises a silver electrode per se without any surface treatment for forming a silver chloride film. Since the present apparatus is used for the measurement of chlorine concentration, a silver chloride film is formed on the silver electrode surface due to chloride ion originally contained in the sample solution and the same can be used as the reference electrode.

Figure 20 shows results of an experiment in which a silver electrode was used as the reference electrode and the temperature coefficient (chlorine concentration) was determined as in Figure 16. Although slight differences were observed in the current sensitivity, almost identical results were obtained.

The present invention is not limited to the embodiments above. Those skilled in the art can make modifications in the measurement procedures and modify the apparatuses, and can make various modifications and changes without departing from the spirit of the present invention.

Reference Signs List

100 RESIDUAL CHLORINE MEASUREMENT APPARATUS
101 SAMPLE SOLUTION
102 WORKING ELECTRODE
103 REFERENCE ELECTRODE
104 COUNTER ELECTRODE
105 POTENTIOSTAT
106 INFORMATION PROCESSING DEVICE (FIRST INFORMATION PROCESSING DEVICE)
107 CELL
110 WIRING
120 TEMPERATURE MEASURING UNIT
130 pH MEASURING UNIT
140 SECOND INFORMATION PROCESSING DEVICE
200 RESIDUAL CHLORINE MEASUREMENT APPARATUS FOR FIA
207 FLOW CELL
208 SOLUTION TANK
209 PUMP
211 FLOW TUBE
212 FLOW PATH
213 INFLOW PORT
214 OUTFLOW PORT
220 TEMPERATURE MEASURING UNIT
230 pH MEASURING UNIT

Each of the publications, patents and patent applications referred to in the present specification are incorporated by reference into the present specification.

## Claims

1. A method for measuring the residual chlorine concentration in a sample solution possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein the method comprises:

(i) measuring the current value when the potential of the conductive diamond electrode against the silver/silver chloride electrode is set to a given potential in the range of from 0 V to +1.6 V and calculating the residual chlorine concentration based on hypochlorite ion;
(ii) measuring the current value when the potential of the conductive diamond electrode against the silver/silver chloride electrode is set to a given potential in the range of from +0.4 V to -1.0 V and calculating the residual chlorine concentration based on hypochlorous acid; and
(iii) adding the residual chlorine concentration based on hypochlorite ion calculated in step (i) above and the residual chlorine concentration based on hypochlorous acid calculated in step (ii) above, designating said total residual chlorine concentration obtained by the addition as the residual chlorine concentration of the sample solution.

**2.** The method of claim 1, further comprising, after step (iii),

(iv) bringing a temperature measuring unit into contact with the sample solution and measuring the solution temperature of the sample solution with said temperature measuring unit, and calculating the temperature correction value from the measured solution temperature; and
(v) carrying out correction on said total residual chlorine concentration obtained by the addition according to claim 1 based on the temperature correction value in step (iv), and designating the total residual chlorine concentration after the correction as the residual chlorine concentration of the sample solution.

**3.** A method for measuring the pH of a sample solution possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein the method comprises:

(i) measuring the current value when the potential of the conductive diamond electrode against the silver/silver chloride electrode is set to a given potential in the range of from 0 V to +1.6 V and calculating the residual chlorine concentration based on hypochlorite ion;
(ii) measuring the current value when the potential of the conductive diamond electrode against the silver/silver chloride electrode is set to a given potential in the range of from +0.4 V to -1.0 V and calculating the residual chlorine concentration based on hypochlorous acid;
(iii) calculating the compositional ratio of hypochlorite ion and hypochlorous acid by comparing the residual chlorine concentration based on hypochlorite ion calculated in step (i) and the residual chlorine concentration based on hypochlorous acid calculated in step (ii); and
(iv) calculating pH by applying the calculated compositional ratio to an effective chlorine compositional ratio curve, designating said calculated pH as the pH of the sample solution.

**4.** The method of claim 3, further comprising, after step (iv),

(v) bringing a temperature measuring unit into contact with the sample solution and measuring the solution temperature of the sample solution with said temperature measuring unit, and calculating the temperature correction value from the measured solution temperature; and
(vi) carrying out correction on the calculated pH according to claim 3 based on the temperature correction value in step (v), designating said pH of the sample solution after the correction as the pH of the sample solution.

**5.** A method for automatic diagnosis of a measuring instrument, further comprising, after step (iv),

(v) bringing a pH measuring unit into contact with the sample solution, and measuring the pH of the sample solution by the pH measuring unit; and
(vi) comparing the pH calculated from the value of current flowing through the working electrode according to claim 3 with the pH measured by the pH measuring unit in step (v), wherein when the difference therebetween is within a predetermined error, the measuring instrument is determined to be normal.

**6.** The method according to any one of claims 1 to 5, further comprising an electrode initializing step, wherein the electrode initializing step comprises:

repeating the following steps (i) and (ii) as a pair one or more times:

(i) applying a positive or negative first pulse voltage for 0.01 to 60 sec; and
(ii) applying a negative or positive second pulse voltage, said second pulse voltage having a sign reverse to the pulse voltage applied in step (i), for 0.01 to 60 sec.

**7.** A method for measuring the residual chlorine concentration in a sample solution having a known pH and possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein, when the pH of the sample solution is 7.5 or lower, the method comprises:

measuring the current value when the potential of the conductive diamond electrode against the silver/silver chloride electrode is set to a given potential in the range of from +0.4 V to -1.0 V and calculating the concentration of hypochlorous acid; and
designating the residual chlorine concentration calculated by applying the pH of the sample solution and the calculated hypochlorous acid concentration to an effective chlorine compositional ratio curve, as the residual chlorine concentration of the sample solution.

**8.** The method of claim 7, wherein the pH of the sample solution is 4 to 7.5.

**9.** A method for measuring the residual chlorine concentration in a sample solution having a known pH and possibly containing residual chlorine, by bringing a working electrode, a counter electrode and a reference electrode into contact with the sample solution, applying a voltage between the working electrode and the reference electrode, and measuring the value of current flowing through the working electrode under the voltage,
wherein the working electrode is a boron doped conductive diamond electrode; and
the reference electrode is a silver/silver chloride electrode,
wherein, when the pH of the sample solution is 7.5 or higher, the method comprises:

measuring a current value when the potential of the conductive diamond electrode against the silver/silver chloride electrode is set to a given potential in the range of from 0 V to +1.6 V and calculating the concentration of hypochlorite ion; and
designating the residual chlorine concentration calculated by applying the pH of the sample solution and the calculated hypochlorite ion concentration to an effective chlorine compositional ratio curve, as the residual chlorine concentration of the sample solution.

**10.** The method of claim 9, wherein the pH of the sample solution is higher than 7.5 and 10 or lower.

**11.** The method according to any one of claims 1 to 10, wherein the measurement is carried out continuously by a flow injection method.

**12.** The method of claim 11, wherein the measurement is carried out at a constant potential.

**13.** A continuous measuring method, comprising repeating the following steps (a) and (b) as a pair one or more times:

(a) carrying out the electrode initializing step according to claim 6 before measurement; and then
(b) carrying out the constant-potential measurement according to claim 12.

**14.** A residual chlorine measurement apparatus for measuring the residual chlorine concentration in a sample solution, said apparatus comprising:
a working electrode; a counter electrode; a reference electrode; a voltage applying unit for applying a voltage between the working electrode and the reference electrode; a current measuring unit for measuring the value of current flowing through the working electrode under the applied voltage; and an information processing device for calculating the residual chlorine concentration based on a current measurement signal from the current measuring unit, wherein the working electrode is a boron doped conductive diamond electrode;
the reference electrode is a silver/silver chloride electrode; and

the information processing device

(i) measures the current value by controlling the potential of the conductive diamond electrode against the silver/silver chloride electrode at a given potential in the range of from 0 V to +1.6 V;
(ii) measures the current value by controlling the potential of the conductive diamond electrode against the silver/silver chloride electrode at a given potential in the range of from +0.4 V to -1.0 V; and
(iii) calculates the residual chlorine concentration based on hypochlorite ion from the current value measured in step (i), calculates the residual chlorine concentration based on hypochlorous acid from the current value measured in step (ii), designating the total residual chlorine concentration obtained by adding the calculated residual chlorine concentration based on hypochlorite ion and the calculated residual chlorine concentration based on hypochlorous acid, as the residual chlorine concentration of the sample solution,

wherein the measurement in step (i) and the measurement in step (ii) can be carried out successively in any order, or simultaneously.

**15.** The apparatus of claim 14, further comprising: a temperature measuring unit for measuring the temperature of the sample solution; and a second information processing device for calculating the temperature of the sample solution based on the temperature measurement signal from the temperature measuring unit,
wherein, after step (iii), the apparatus

(iv) brings the temperature measuring unit into contact with the sample solution, measures the solution temperature of the sample solution with said temperature measuring unit, and calculates a temperature correction value from the measured solution temperature; and
(v) carries out correction on the total residual chlorine concentration obtained by the addition according to claim 14 based on the temperature correction value in step (iv), and designates the total residual chlorine concentration after the correction as the residual chlorine concentration of the sample solution.

**16.** An apparatus for measuring the pH of a sample solution possibly containing residual chlorine, said apparatus comprising:

a working electrode; a counter electrode; a reference electrode; a voltage applying unit for applying a voltage between the working electrode and the reference electrode; a current measuring unit for measuring the value of current flowing through the working electrode under the applied voltage; and an information processing device for calculating the residual chlorine concentration based on a current measurement signal from the current measuring unit, wherein the working electrode is a boron doped conductive diamond electrode;
the reference electrode is a silver/silver chloride electrode; and
wherein the information processing device

(i) measures the current value by controlling the potential of the conductive diamond electrode against the silver/silver chloride electrode at a given potential in the range of from 0 V to +1.6 V;
(ii) measures the current value by controlling the potential of the conductive diamond electrode against the silver/silver chloride electrode at a given potential in the range of from +0.4 V to -1.0 V; and
(iii) calculates the residual chlorine concentration based on hypochlorite ion from the current value measured in step (i), calculates the residual chlorine concentration based on hypochlorous acid from the current value measured in step (ii), and calculating the compositional ratio of hypochlorite ion and hypochlorous acid by comparing the residual chlorine concentration calculated in step (i) and the residual chlorine concentration calculated in step (ii); and
(iv) calculates a pH by applying the calculated compositional ratio to an effective chlorine compositional ratio curve, and designates the calculated pH as the pH of the sample solution, wherein the measurement in step (i) and the measurement in step (ii) can be carried out successively in any order, or simultaneously.

**17.** The apparatus of claim 16, further comprising: a temperature measuring unit for measuring the temperature of the sample solution; and a second information processing device for calculating the temperature of the sample solution based on the temperature measurement signal from the temperature measuring unit,
wherein, after step (iv), the apparatus

(v) brings the temperature measuring unit into contact with the sample solution, measures the solution temperature of the sample solution with said temperature measuring unit, and calculates a temperature correction value from the measured solution temperature; and
(vi) carries out correction on the calculated pH according to claim 16 based on the temperature correction value in step (v), and designates the pH of the sample solution after the correction as the pH of the sample solution.

**18.** The apparatus of claim 16, further comprising: a pH measuring unit for measuring the pH of a sample solution; and a second information processing device for calculating the pH of the sample solution based on a pH measurement signal from the pH measuring unit,
wherein the apparatus further comprises an automatically diagnosing function of the following (v) and (vi) after step (iv),

(v) bringing the pH measuring unit into contact with the sample solution, and measuring the pH of the sample solution by the pH measuring unit; and
(vi) comparing the pH calculated from the value of current flowing through the working electrode according to claim 16 with the pH measured by the pH measuring unit in step (v), wherein when the difference therebetween is within a predetermined error, the measuring instrument is determined to be normal.

**19.** The apparatus of any one of claims 14 to 18, comprising the temperature measuring unit of claim 15 or 17, and the pH measuring unit of claim 18.

**20.** The apparatus according to any one of claims 14 to 19, comprising a bipotentiostat and two working electrodes, wherein the measurement in step (i) and the measurement in step (ii) can be carried out simultaneously.

**21.** The apparatus according to any one of claims 14 to 19, comprising two working electrodes, two counter electrodes and two reference electrodes, wherein the measurement in step (i) and the measurement in step (ii) can be carried out simultaneously.

**22.** The apparatus according to any one of claims 14 to 21 for flow injection analysis, further comprising a flow cell, wherein the flow cell comprises the working electrode(s), reference electrode(s) and counter electrode(s) built-in, and comprises a flow tube for passing the sample solution, wherein the working electrode(s), the reference electrode(s) and the counter electrode(s) are arranged in the flow cell such that when the sample solution passes through the flow tube in the flow cell, the sample solution can contact with the working electrode(s), the reference electrode(s) and the counter electrode(s).

**23.** The apparatus of claim 22, wherein the flow cell further comprises a temperature measuring unit and/or pH measuring unit built-in; and the working electrode(s), the reference electrode(s) and the counter electrode(s), and the temperature measuring unit and/or the pH measuring unit are arranged in the flow cell such that when the sample solution passes through the flow tube in the flow cell, the sample solution can further contact with the temperature measuring unit and/or the pH measuring unit.

**24.** The apparatus according to any one of claims 14 to 23, wherein the reference electrode(s) is a silver electrode.

**25.** The apparatus according to any one of claims 14 to 24, wherein the counter electrode(s) is a boron doped conductive diamond electrode.

**26.** The apparatus according to any one of claims 14 to 25, wherein the apparatus further carries out, as an electrode initializing step, said electrode initialization step comprising:
repeating the following steps (i) and (ii) as a pair one or more times:

(i) applying a positive or negative first pulse voltage for 0.01 to 60 sec; and
(ii) applying a negative or positive second pulse voltage, said second pulse voltage having a sign reverse to the pulse voltage applied in step (i), for 0.01 to 60 sec.

Fig. 1

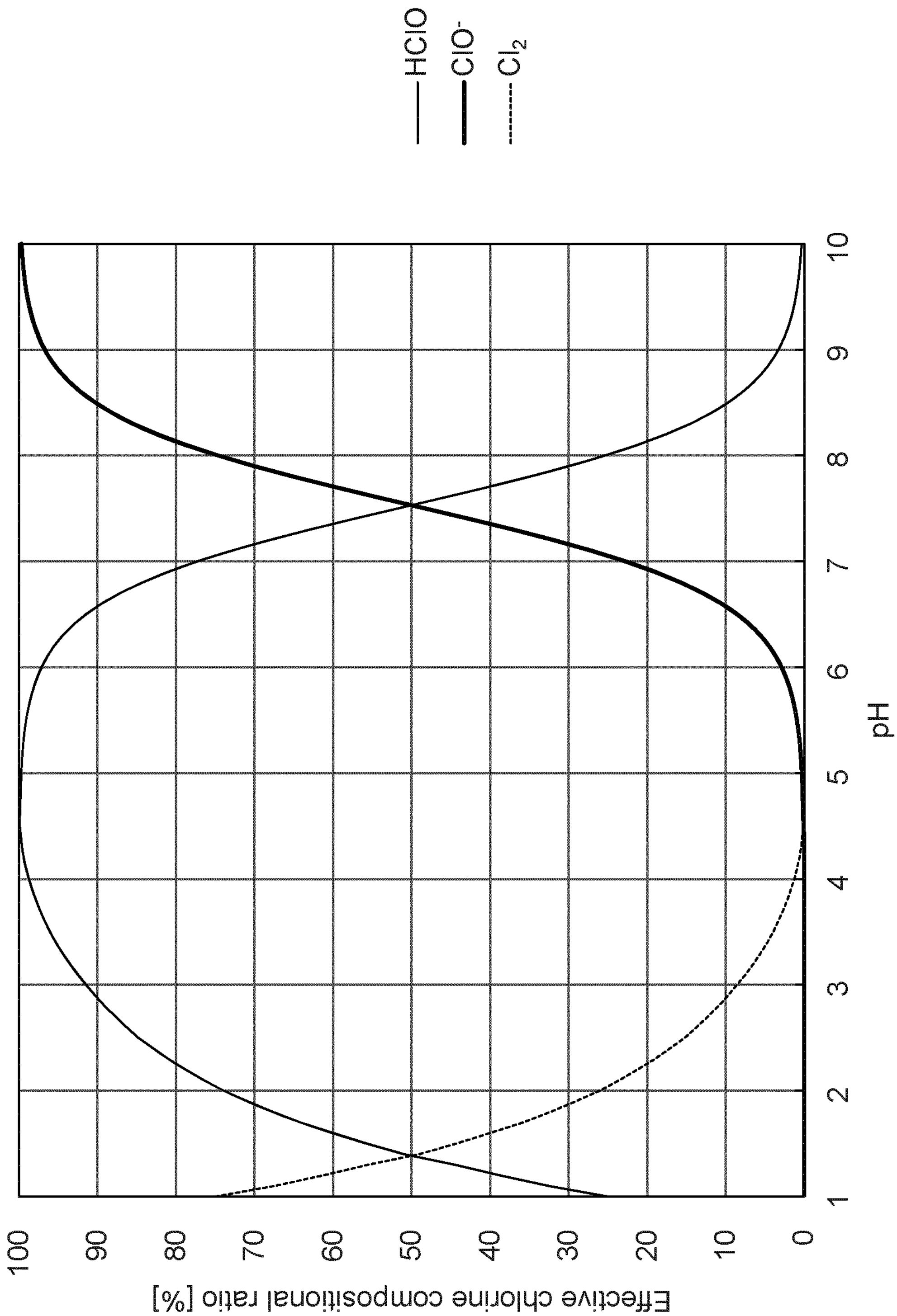
HClO
ClO-
Cl2
Effective chlorine compositional ratio [%]
100
90
80
70
60
50
40
30
20
10
0
1
2
3
4
5
6
7
8
9
10
pH

Fig. 2-1

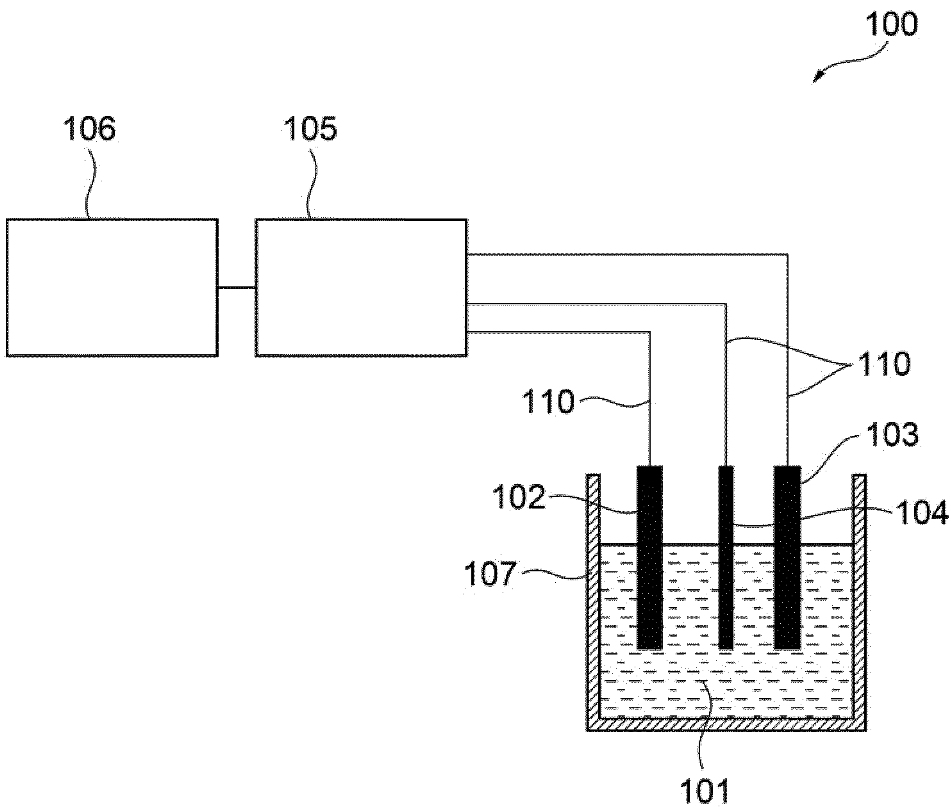

Fig. 2-2

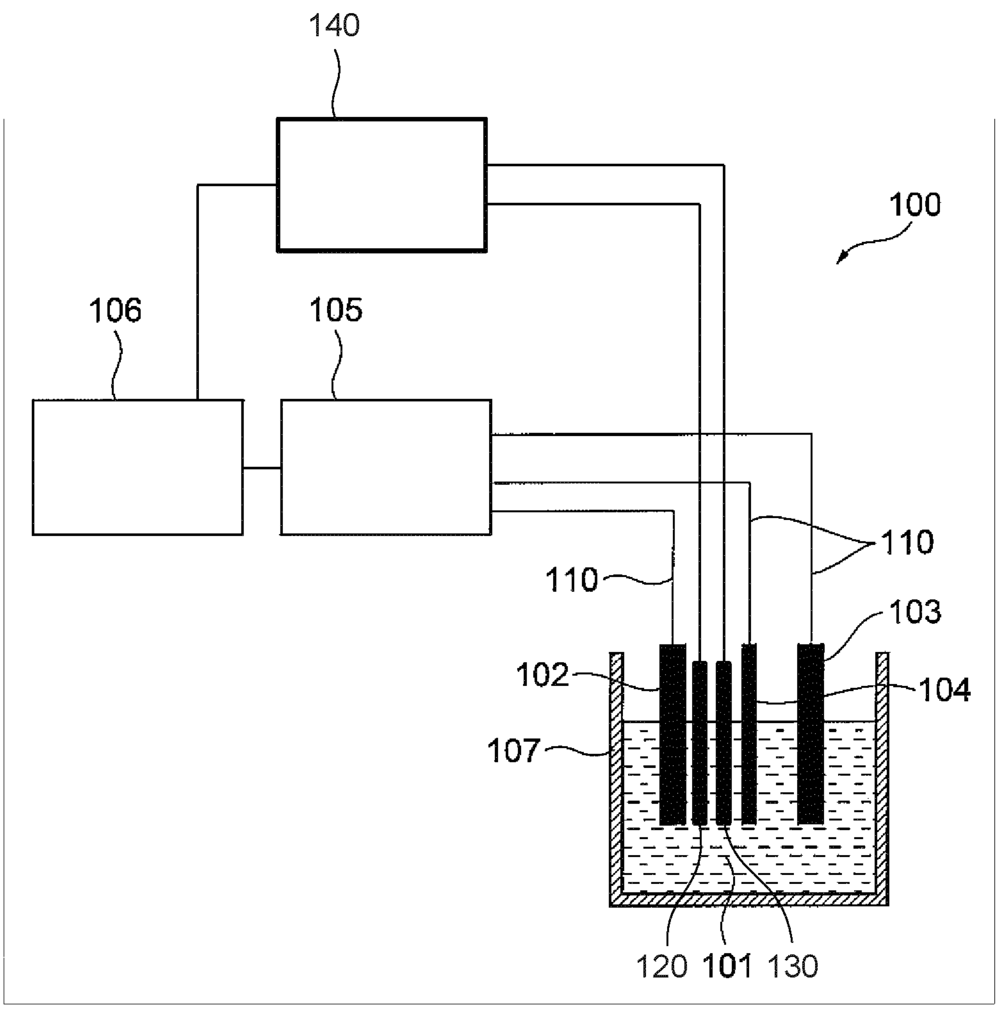

Fig. 3

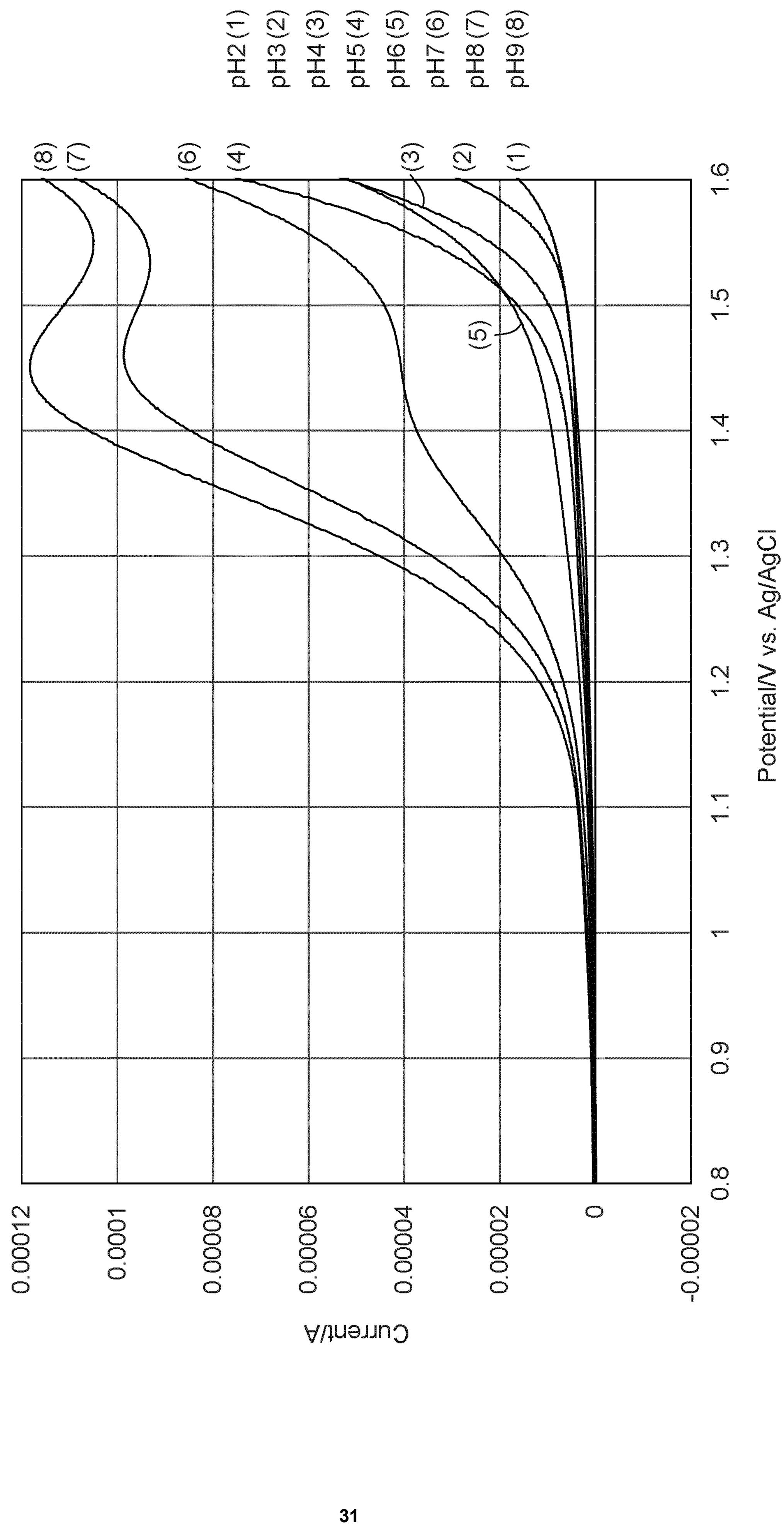
pH2 (1)
pH3 (2)
pH4 (3)
pH5 (4)
pH6 (5)
pH7 (6)
pH8 (7)
pH9 (8)
(8)
(7)
(6)
(4)
(3)
(2)
(1)
(5)
Current/A
0.00012
0.0001
0.00008
0.00006
0.00004
0.00002
0
-0.00002
Potential/V vs. Ag/AgCl
0.8
0.9
1
1.1
1.2
1.3
1.4
1.5
1.6

Fig. 4

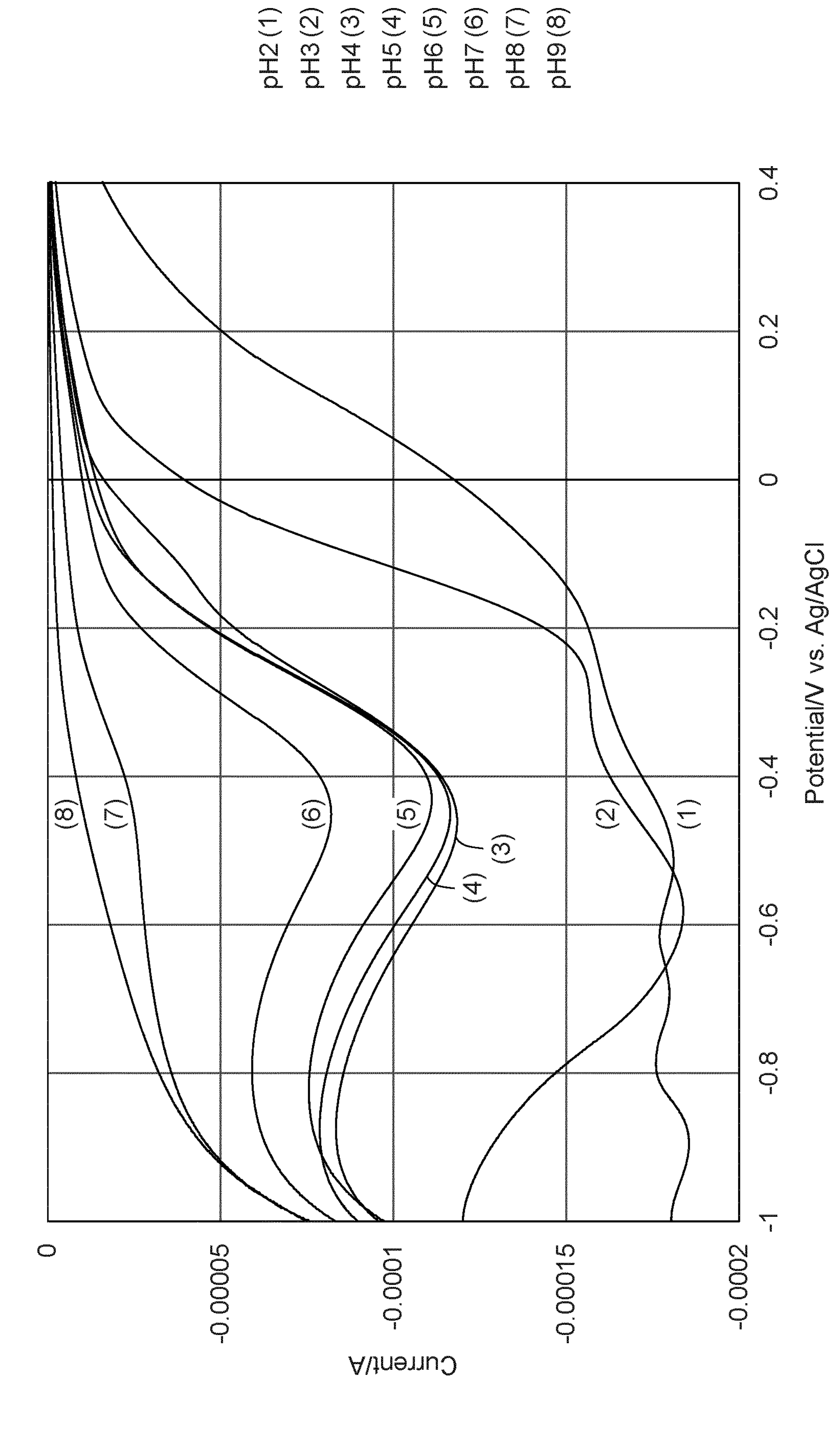
pH2 (1)
pH3 (2)
pH4 (3)
pH5 (4)
pH6 (5)
pH7 (6)
pH8 (7)
pH9 (8)
(1)
(2)
(3)
(4)
(5)
(6)
(7)
(8)
Current/A
0
-0.00005
-0.0001
-0.00015
-0.0002
Potential/V vs. Ag/AgCl
-1
-0.8
-0.6
-0.4
-0.2
0
0.2
0.4

Fig. 5

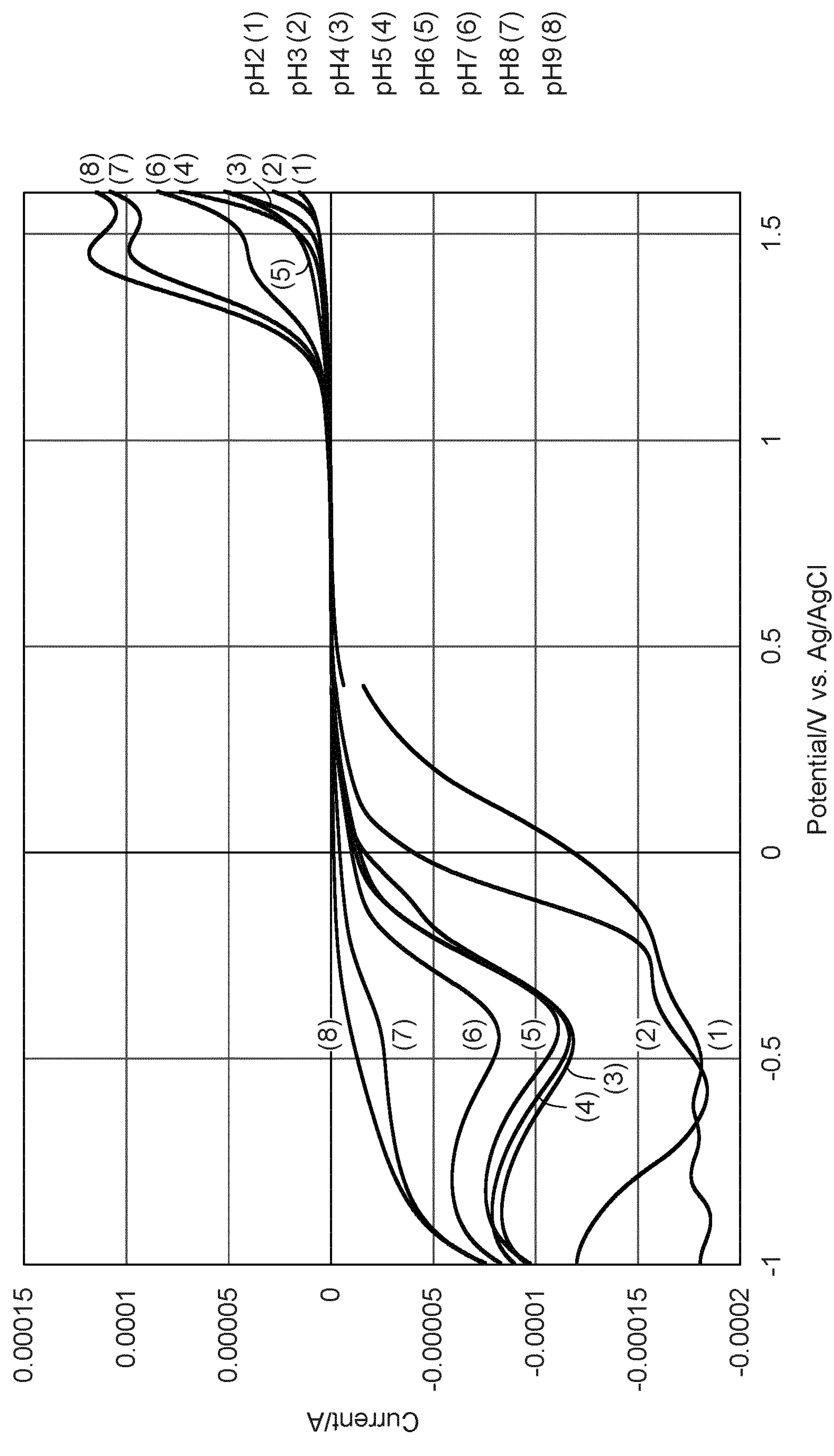
Current/A
0.00015
0.0001
0.00005
0
-0.00005
-0.0001
-0.00015
-0.0002
-1
-0.5
0
0.5
1
1.5
Potential/V vs. Ag/AgCl
pH2 (1)
pH3 (2)
pH4 (3)
pH5 (4)
pH6 (5)
pH7 (6)
pH8 (7)
pH9 (8)

Fig. 6

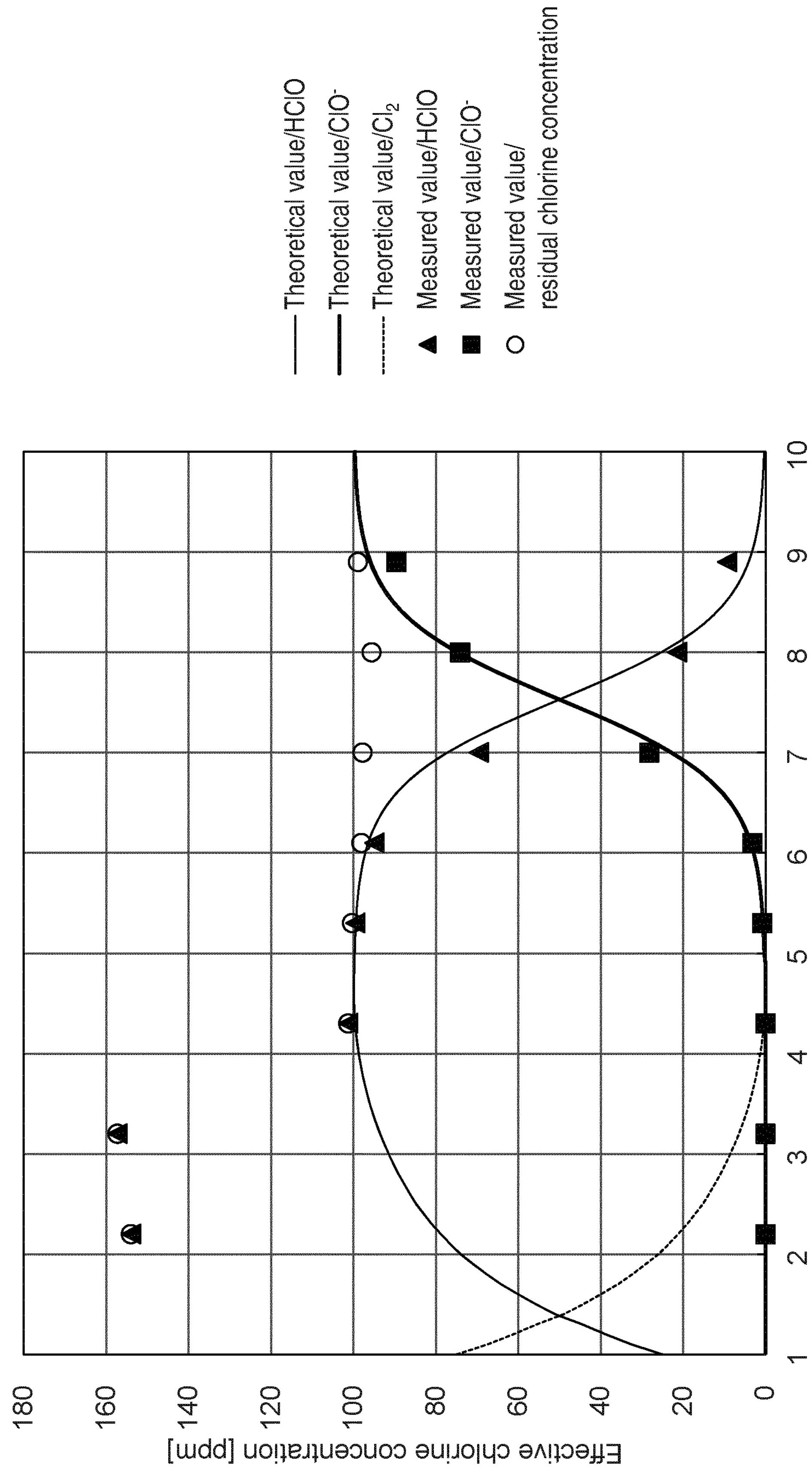

Effective chlorine concentration [ppm]
0
20
40
60
80
100
120
140
160
180
pH
1
2
3
4
5
6
7
8
9
10
Theoretical value/HClO
Theoretical value/ClO-
Theoretical value/Cl2
Measured value/HClO
Measured value/ClO-
Measured value/
residual chlorine concentration

Fig. 7

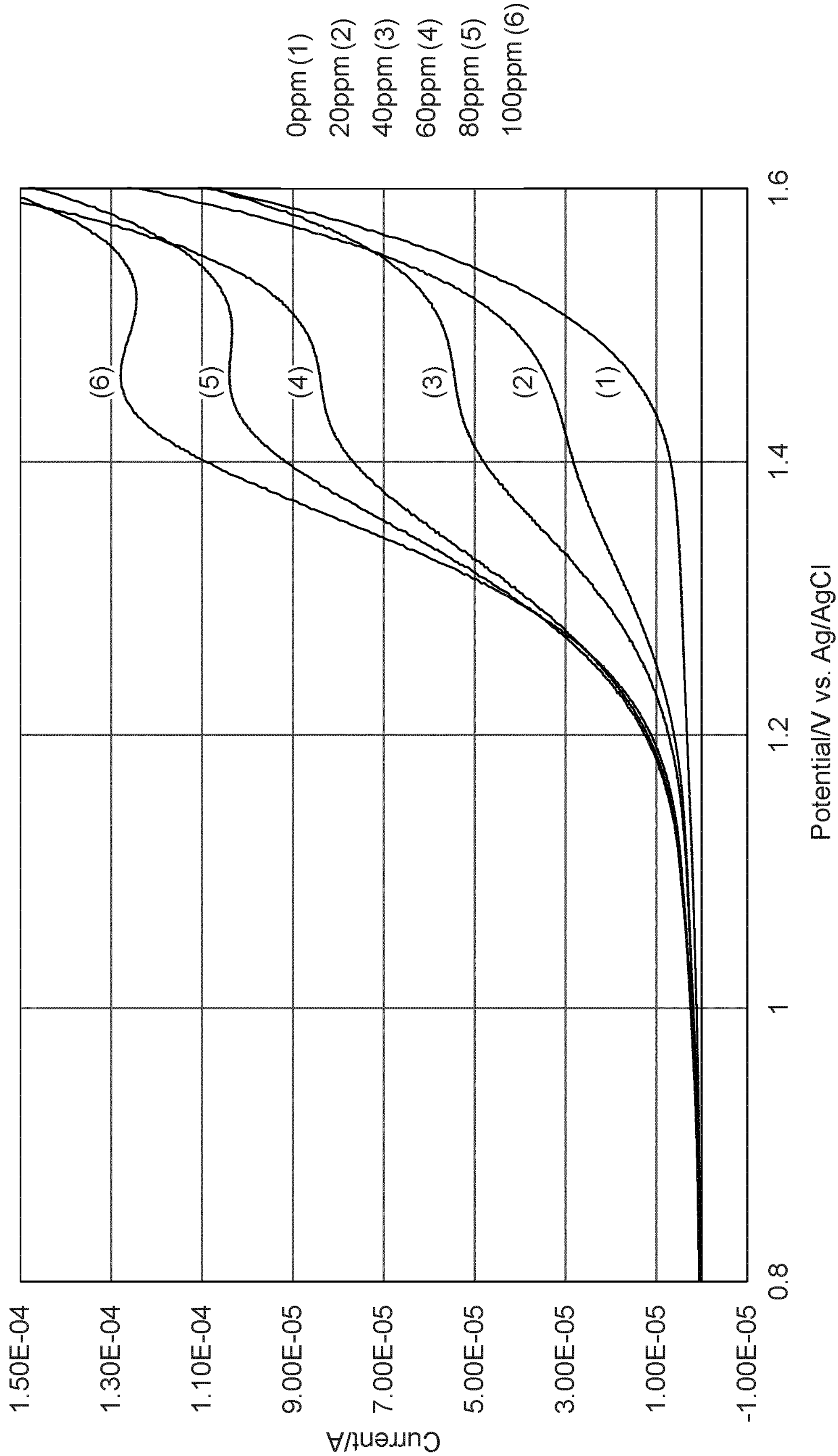
0ppm (1)
20ppm (2)
40ppm (3)
60ppm (4)
80ppm (5)
100ppm (6)
(1)
(2)
(3)
(4)
(5)
(6)
Current/A
1.50E-04
1.30E-04
1.10E-04
9.00E-05
7.00E-05
5.00E-05
3.00E-05
1.00E-05
-1.00E-05
0.8
1
1.2
1.4
1.6
Potential/V vs. Ag/AgCl

Fig. 8

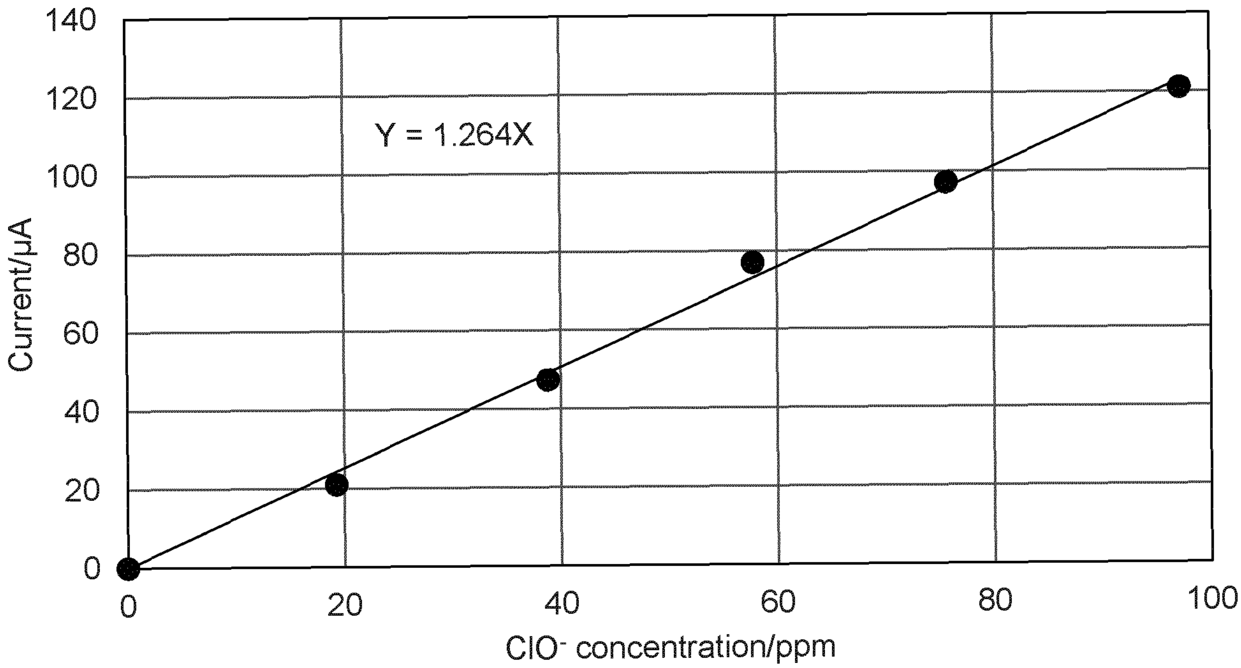
140
120
100
80
60
40
20
0
Current/μA
Y = 1.264X
0
20
40
60
80
100
ClO- concentration/ppm

Fig. 9

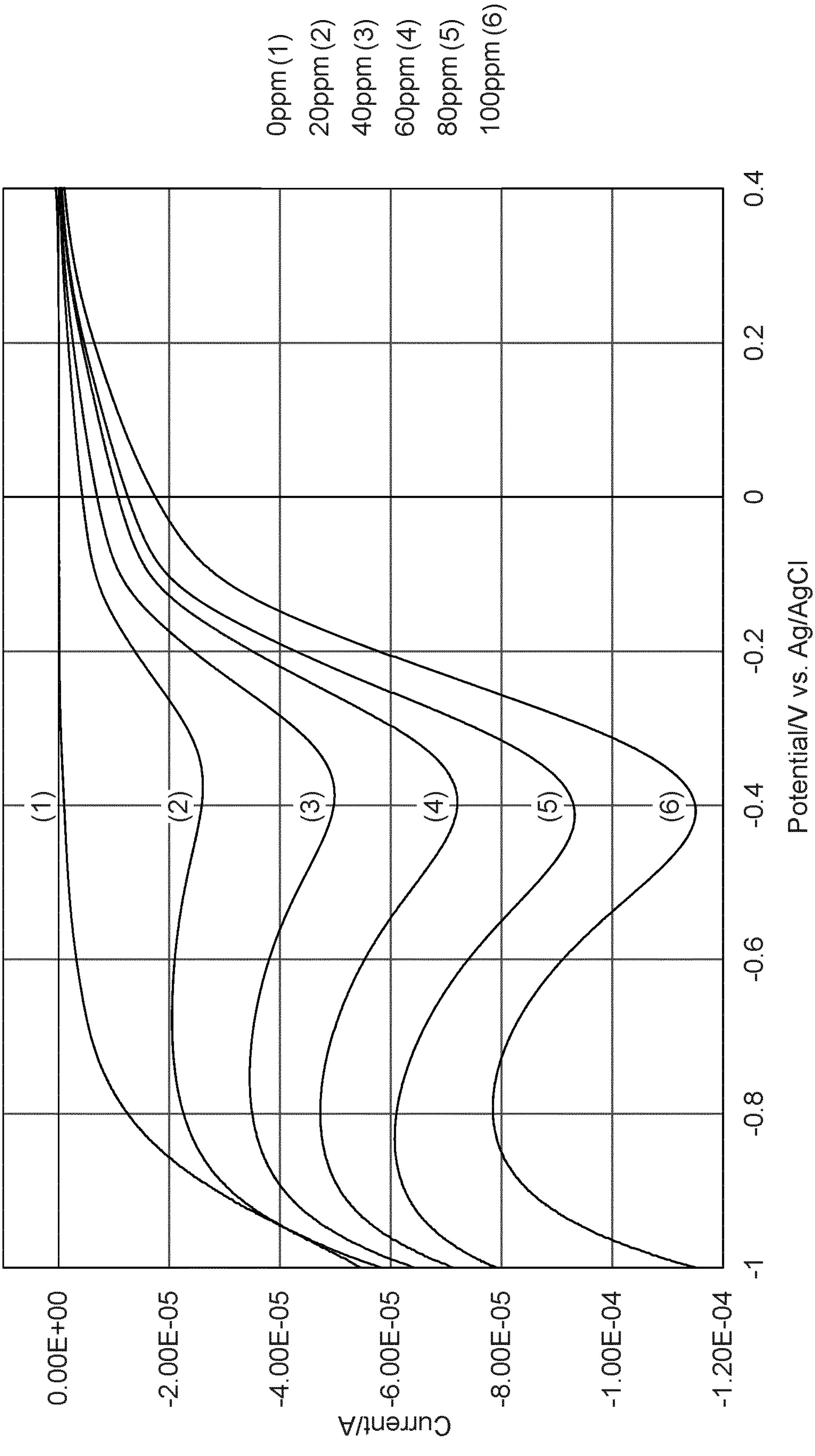
0ppm (1)
20ppm (2)
40ppm (3)
60ppm (4)
80ppm (5)
100ppm (6)
(1)
(2)
(3)
(4)
(5)
(6)
0.00E+00
-2.00E-05
-4.00E-05
-6.00E-05
-8.00E-05
-1.00E-04
-1.20E-04
Current/A
-1
-0.8
-0.6
-0.4
-0.2
0
0.2
0.4
Potential/V vs. Ag/AgCl

Fig. 10

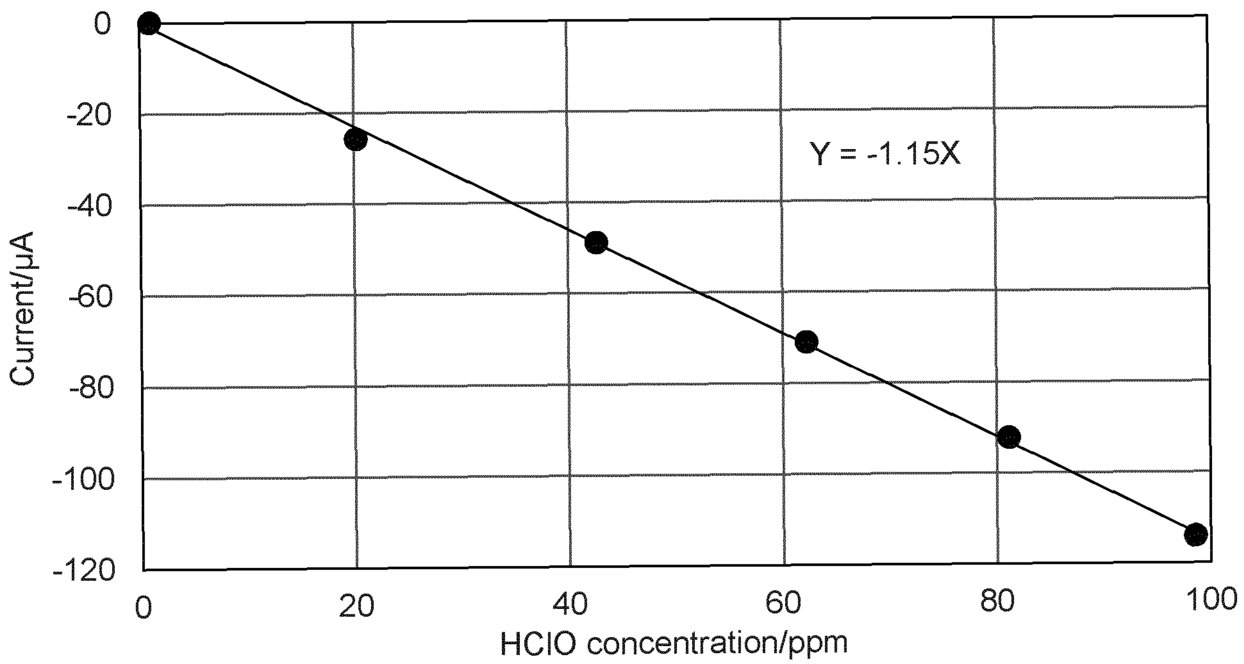
0
-20
-40
-60
-80
-100
-120
Current/μA
Y = -1.15X
0
20
40
60
80
100
HClO concentration/ppm

Fig. 11-1

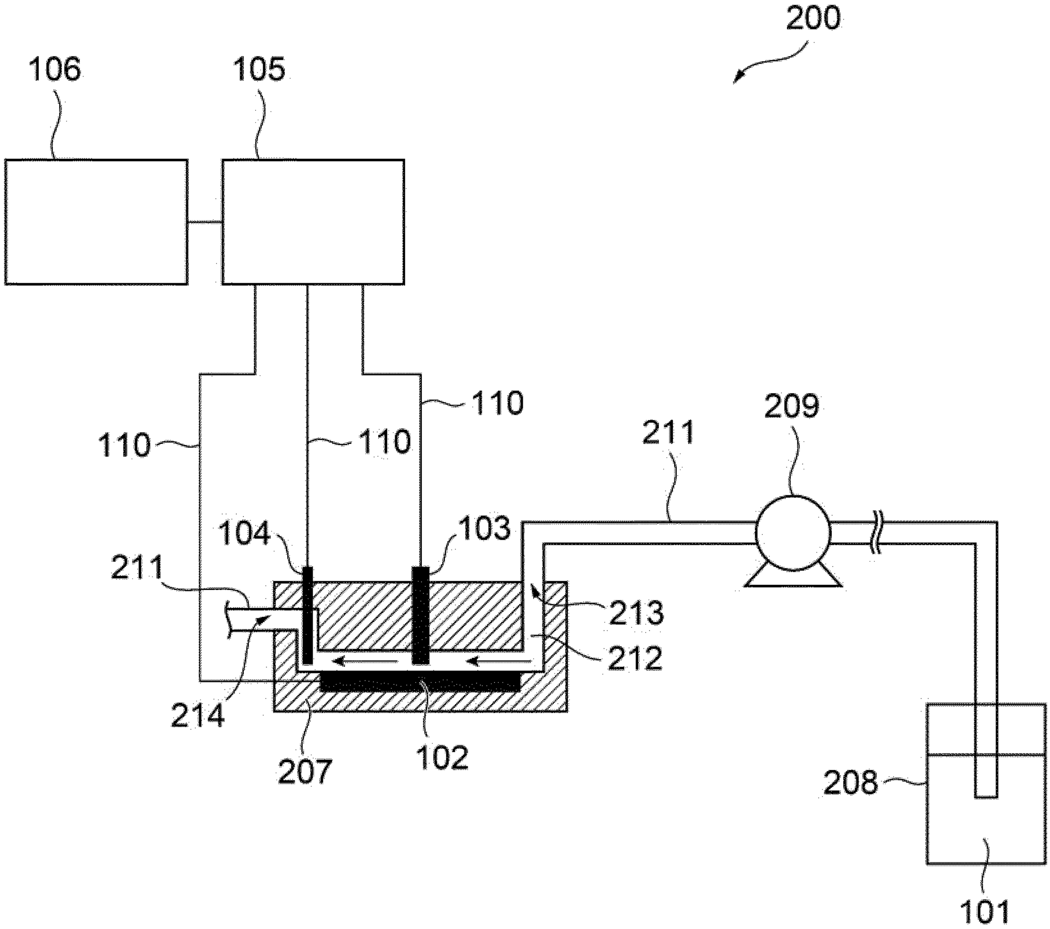
200
106
105
110
110
110
110
211
209
104
103
211
213
212
214
207
102
208
101

Fig. 11-2

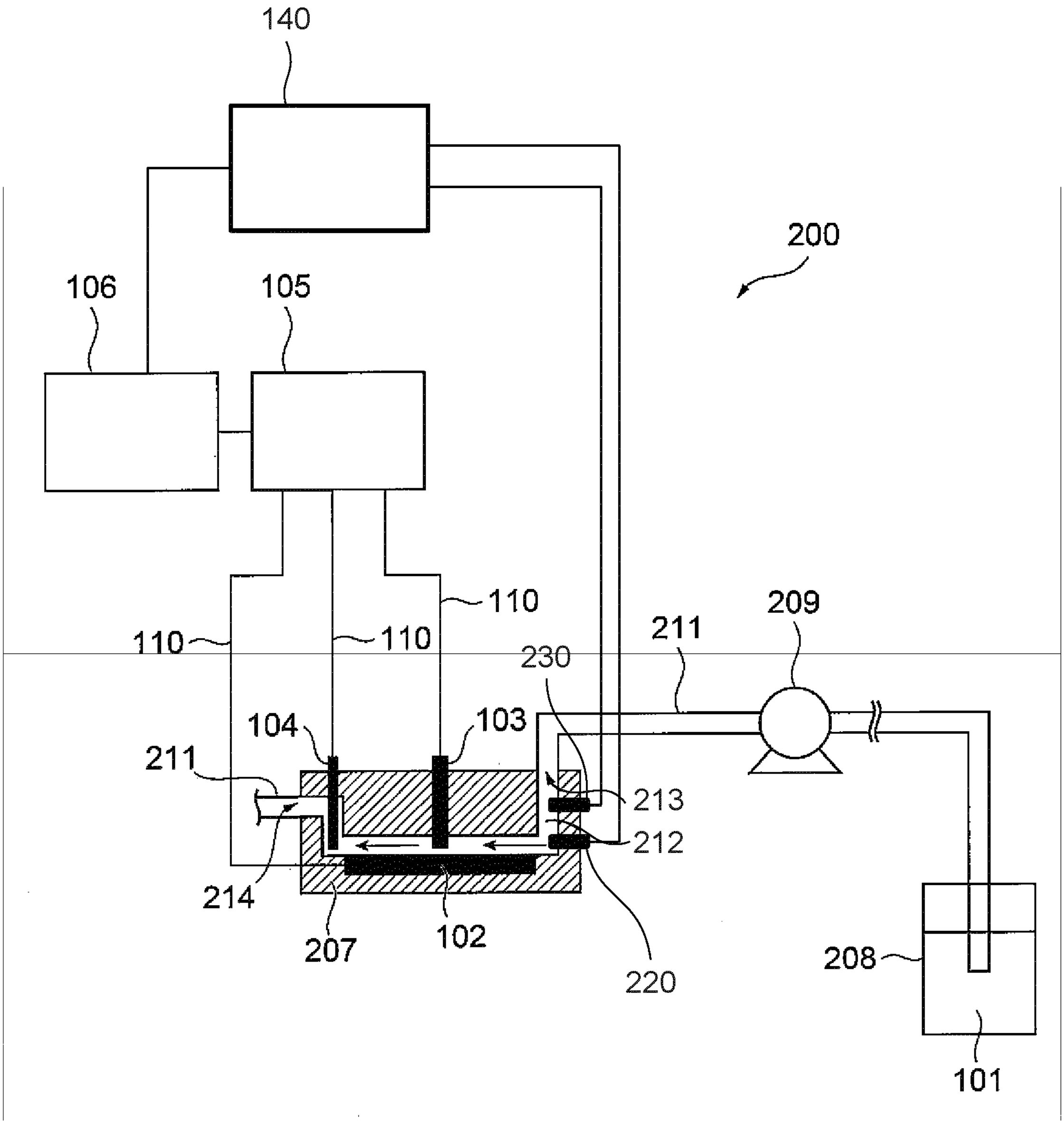
140
200
106
105
110
110
110
211
209
230
104
103
211
213
212
214
207
102
220
208
101

Fig. 12

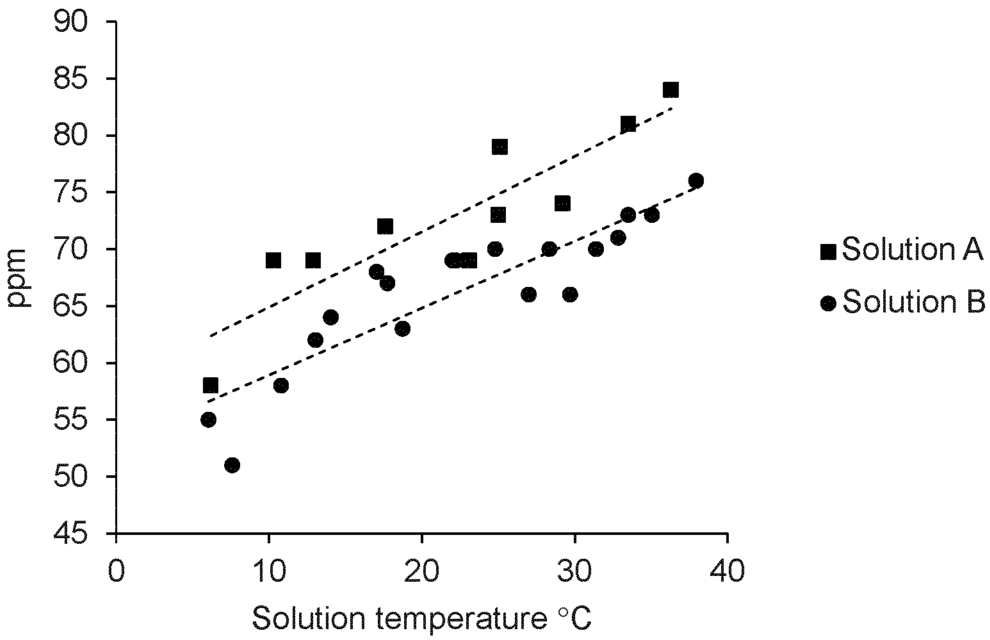
90
85
80
75
70
65
60
55
50
45
ppm
0
10
20
30
40
Solution temperature °C
Solution A
Solution B

Fig. 13

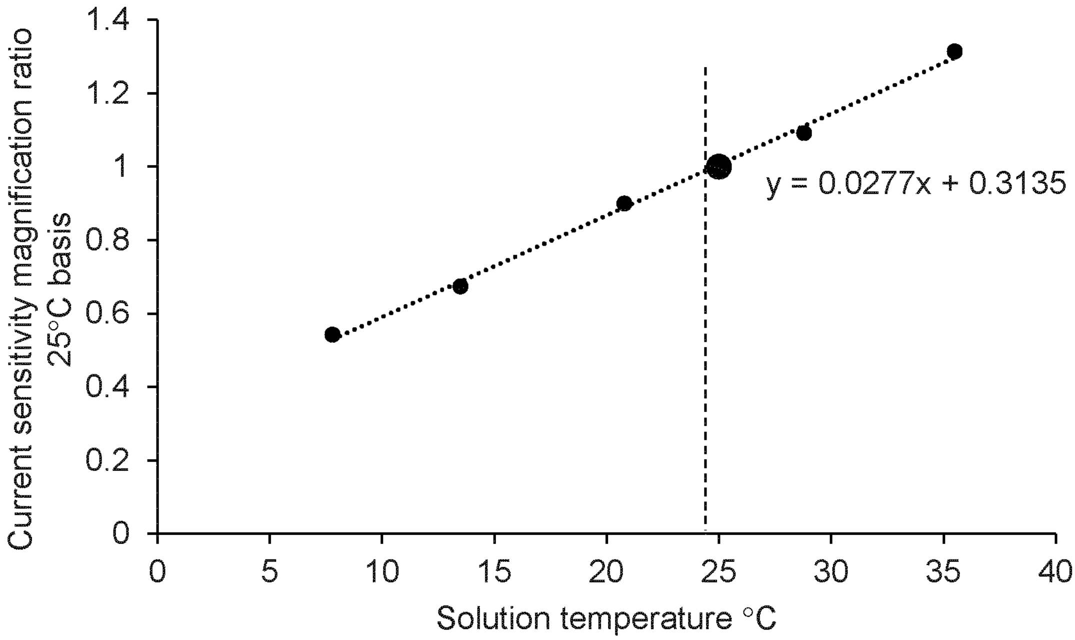
Current sensitivity magnification ratio
25°C basis
1.4
1.2
1
0.8
0.6
0.4
0.2
0
y = 0.0277x + 0.3135
0
5
10
15
20
25
30
35
40
Solution temperature °C

Fig. 14

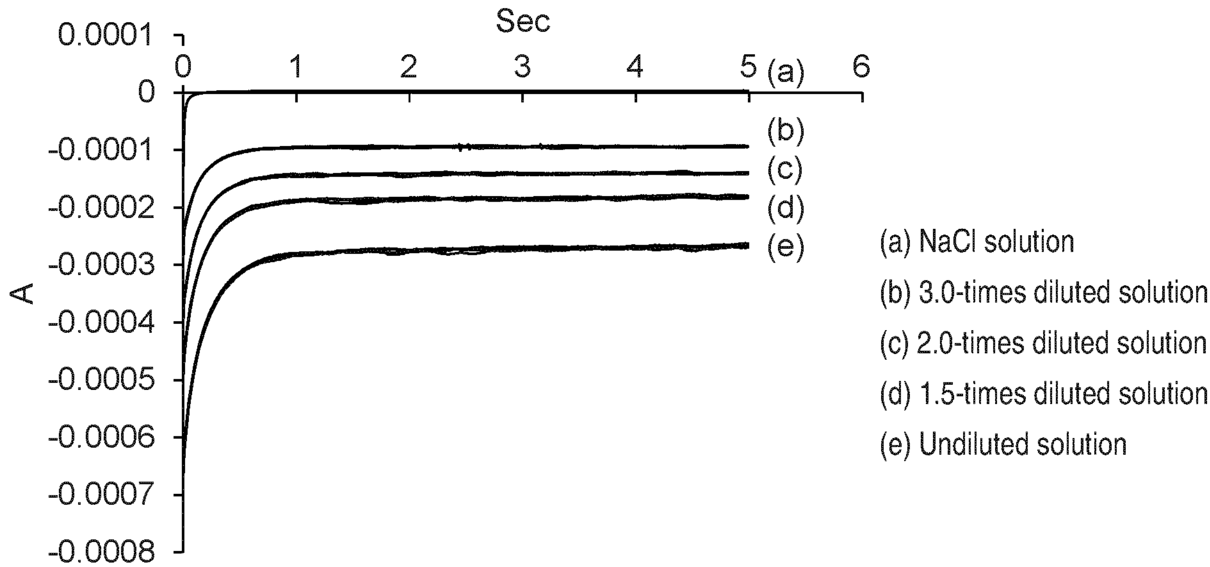
Sec
0.0001
0
-0.0001
-0.0002
-0.0003
-0.0004
-0.0005
-0.0006
-0.0007
-0.0008
A
0
1
2
3
4
5
6
(a)
(b)
(c)
(d)
(e)
(a) NaCl solution
(b) 3.0-times diluted solution
(c) 2.0-times diluted solution
(d) 1.5-times diluted solution
(e) Undiluted solution

Fig. 15

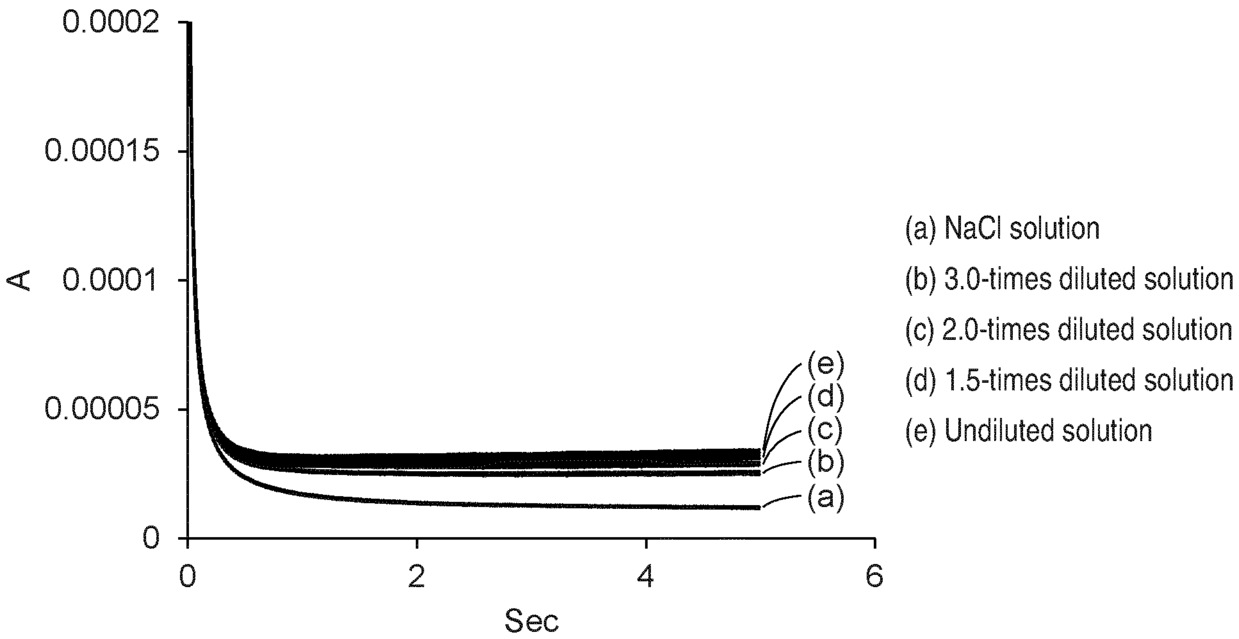
0.0002
0.00015
0.0001
0.00005
0
A
0
2
4
6
Sec
(e)
(d)
(c)
(b)
(a)
(a) NaCl solution
(b) 3.0-times diluted solution
(c) 2.0-times diluted solution
(d) 1.5-times diluted solution
(e) Undiluted solution

Fig. 16

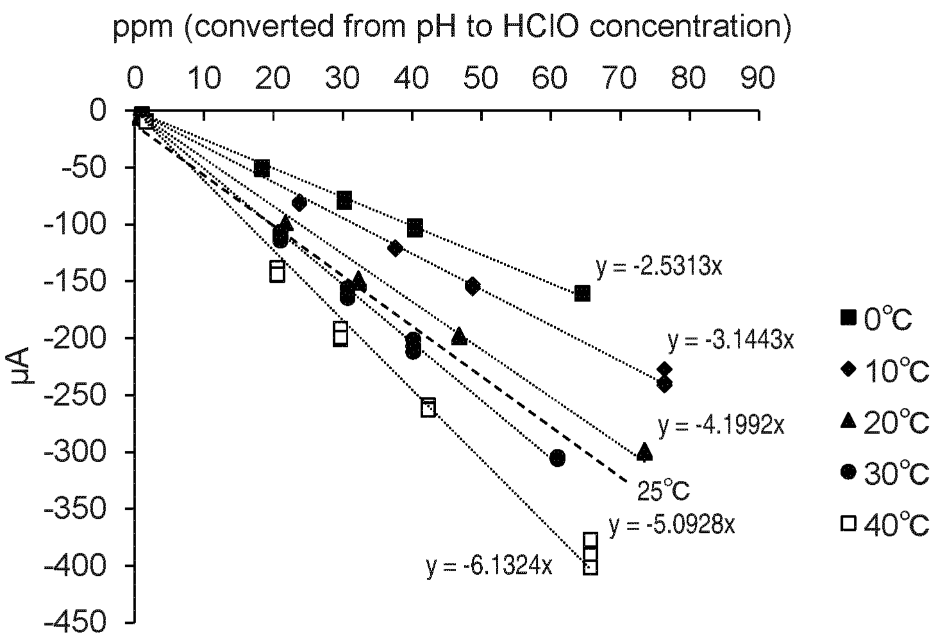
ppm (converted from pH to HClO concentration)
0
10
20
30
40
50
60
70
80
90
0
-50
-100
-150
-200
-250
-300
-350
-400
-450
μA
y = -2.5313x
y = -3.1443x
y = -4.1992x
25°C
y = -5.0928x
y = -6.1324x
0°C
10°C
20°C
30°C
40°C

Fig. 17

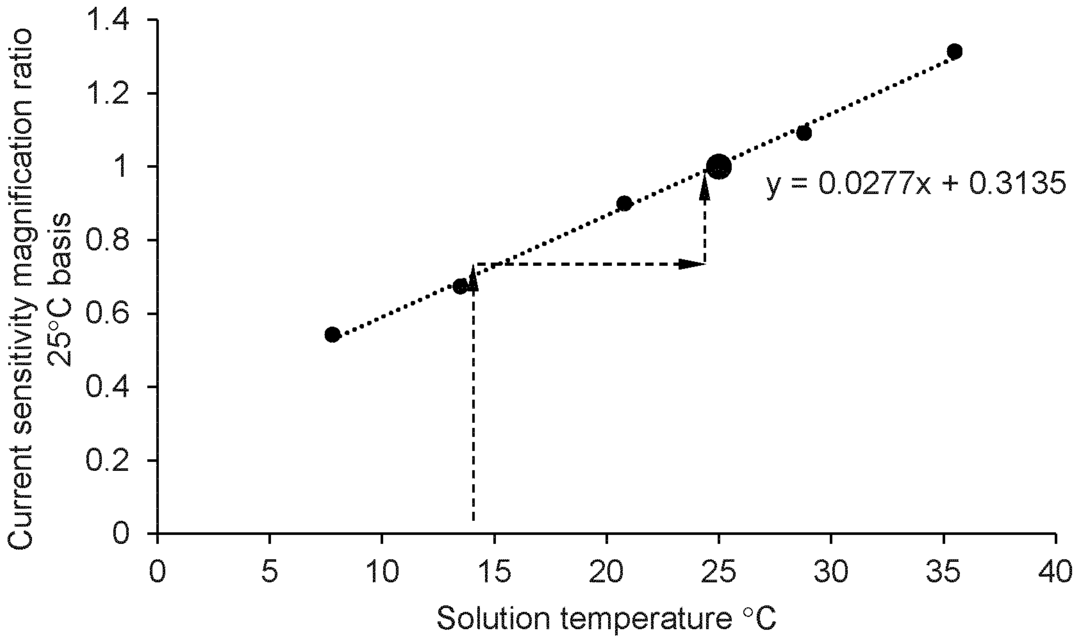
Current sensitivity magnification ratio
25°C basis
1.4
1.2
1
0.8
0.6
0.4
0.2
0
0
5
10
15
20
25
30
35
40
Solution temperature °C
y = 0.0277x + 0.3135

Fig. 18

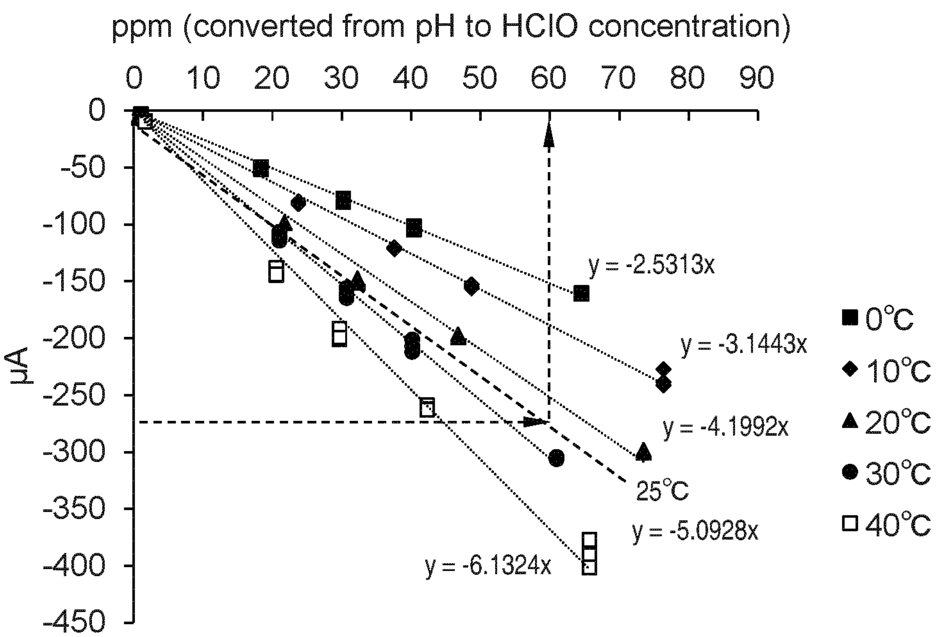
ppm (converted from pH to HClO concentration)
0 10 20 30 40 50 60 70 80 90
0
-50
-100
-150
-200
-250
-300
-350
-400
-450
μA
y = -2.5313x
y = -3.1443x
y = -4.1992x
25℃
y = -5.0928x
y = -6.1324x
0℃
10℃
20℃
30℃
40℃

Fig. 19

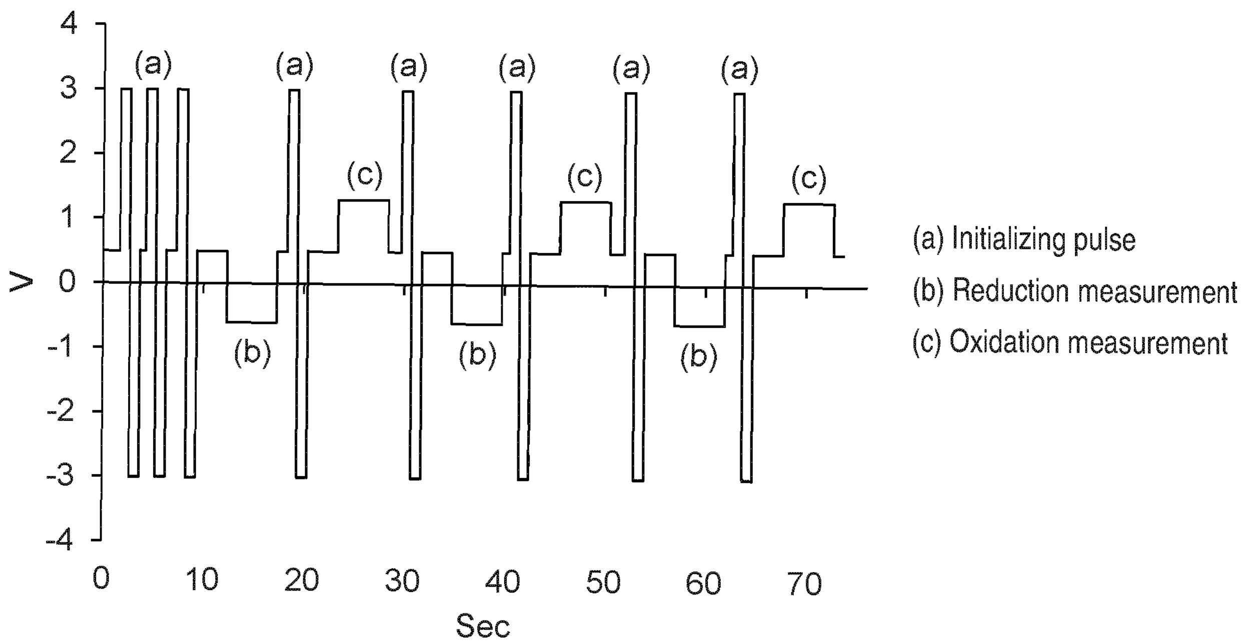
4
3
2
1
0
-1
-2
-3
-4
V
(a)
(b)
(c)
0
10
20
30
40
50
60
70
Sec
(a) Initializing pulse
(b) Reduction measurement
(c) Oxidation measurement

Fig. 20

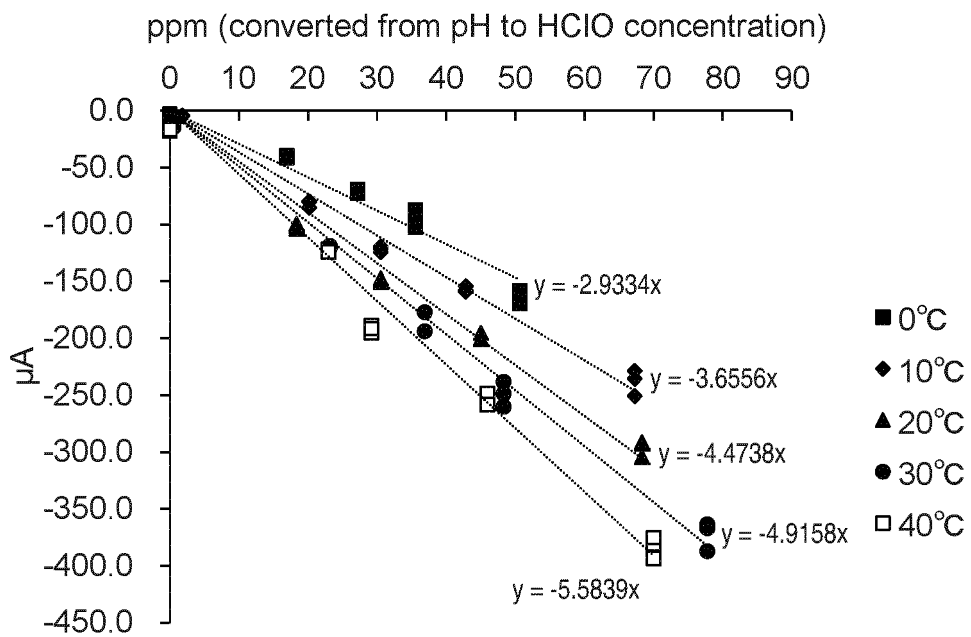
ppm (converted from pH to HClO concentration)
0
10
20
30
40
50
60
70
80
90
0.0
-50.0
-100.0
-150.0
-200.0
-250.0
-300.0
-350.0
-400.0
-450.0
μA
y = -2.9334x
y = -3.6556x
y = -4.4738x
y = -4.9158x
y = -5.5839x
0℃
10℃
20℃
30℃
40℃

Fig. 21-1

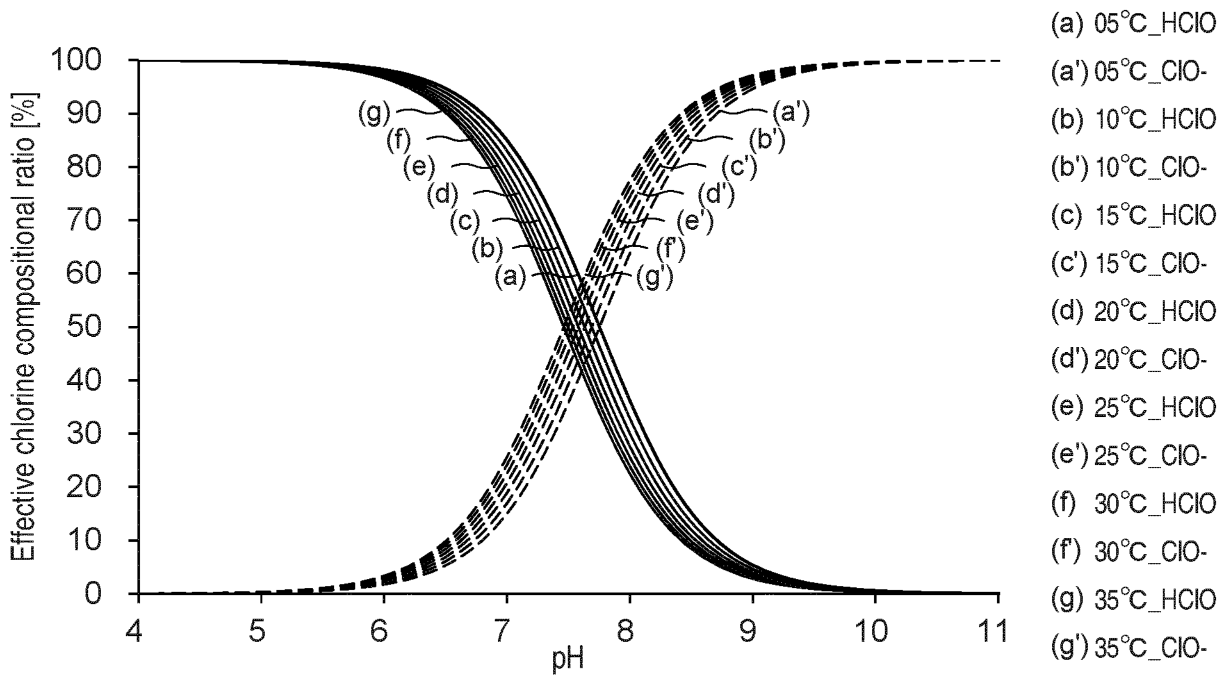
Effective chlorine compositional ratio [%]
100
90
80
70
60
50
40
30
20
10
0
4
5
6
7
8
9
10
11
pH
(a)
(b)
(c)
(d)
(e)
(f)
(g)
(a')
(b')
(c')
(d')
(e')
(f')
(g')
(a) 05°C_HClO
(a') 05°C_ClO-
(b) 10°C_HClO
(b') 10°C_ClO-
(c) 15°C_HClO
(c') 15°C_ClO-
(d) 20°C_HClO
(d') 20°C_ClO-
(e) 25°C_HClO
(e') 25°C_ClO-
(f) 30°C_HClO
(f') 30°C_ClO-
(g) 35°C_HClO
(g') 35°C_ClO-

Fig. 21-2

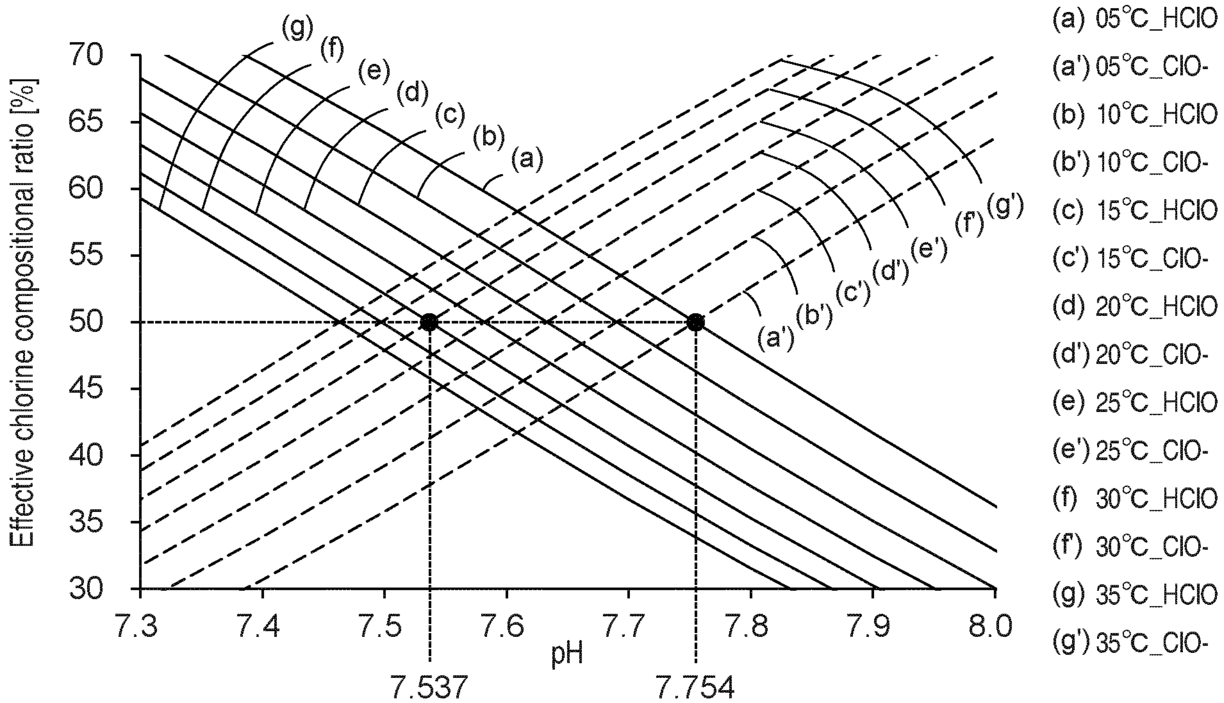

Effective chlorine compositional ratio [%]
70
65
60
55
50
45
40
35
30
7.3
7.4
7.5
7.6
7.7
7.8
7.9
8.0
pH
7.537
7.754
(g)
(f)
(e)
(d)
(c)
(b)
(a)
(a')
(b')
(c')
(d')
(e')
(f')
(g')
(a) 05°C_HClO
(a') 05°C_ClO-
(b) 10°C_HClO
(b') 10°C_ClO-
(c) 15°C_HClO
(c') 15°C_ClO-
(d) 20°C_HClO
(d') 20°C_ClO-
(e) 25°C_HClO
(e') 25°C_ClO-
(f) 30°C_HClO
(f') 30°C_ClO-
(g) 35°C_HClO
(g') 35°C_ClO-

Fig. 22

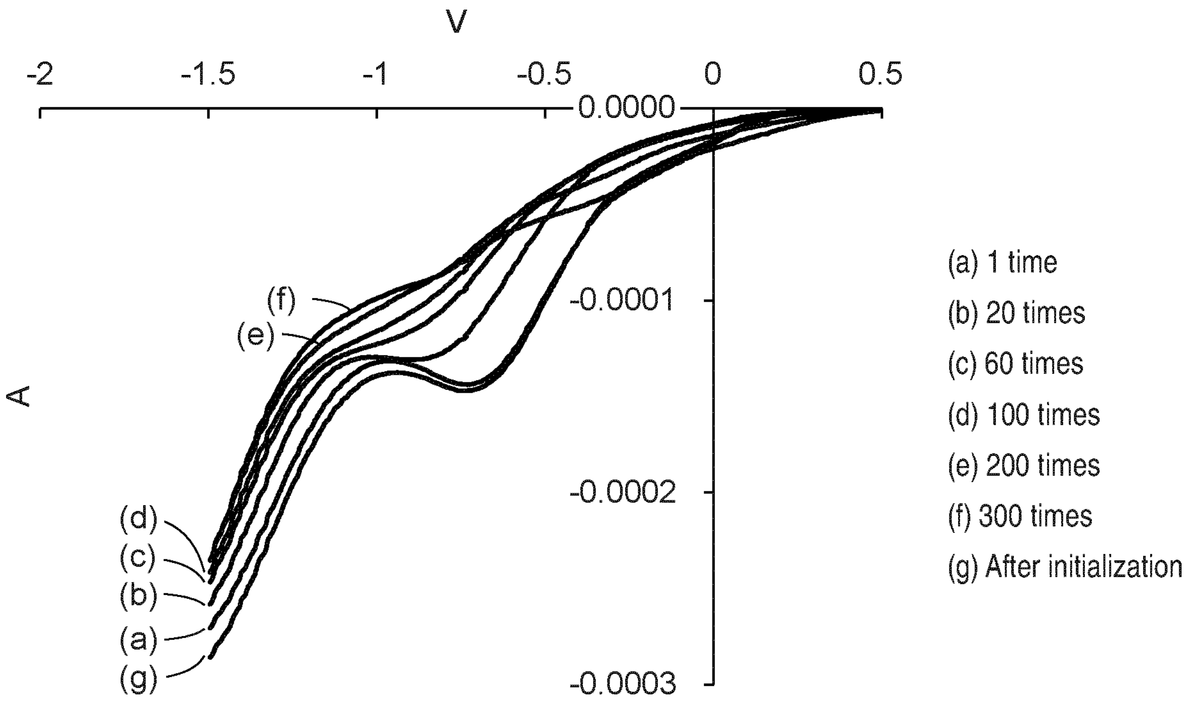
V
-2
-1.5
-1
-0.5
0
0.5
0.0000
-0.0001
-0.0002
-0.0003
A
(a)
(b)
(c)
(d)
(e)
(f)
(g)
(a) 1 time
(b) 20 times
(c) 60 times
(d) 100 times
(e) 200 times
(f) 300 times
(g) After initialization

Fig. 23

A

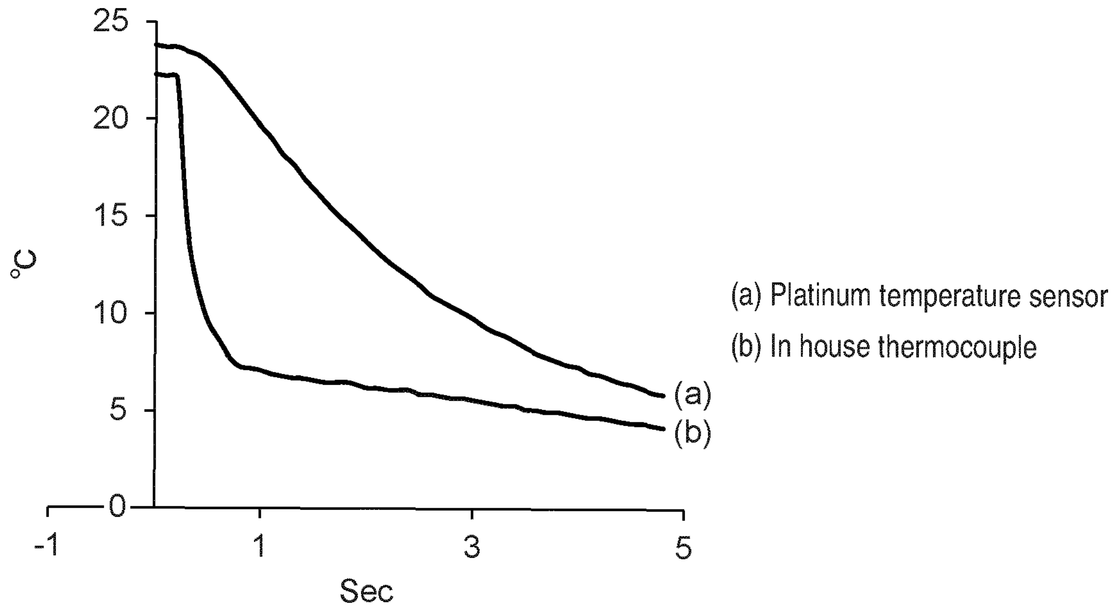
25
20
15
10
5
0
°C
-1
1
3
5
Sec
(a)
(b)
(a) Platinum temperature sensor
(b) In house thermocouple

B

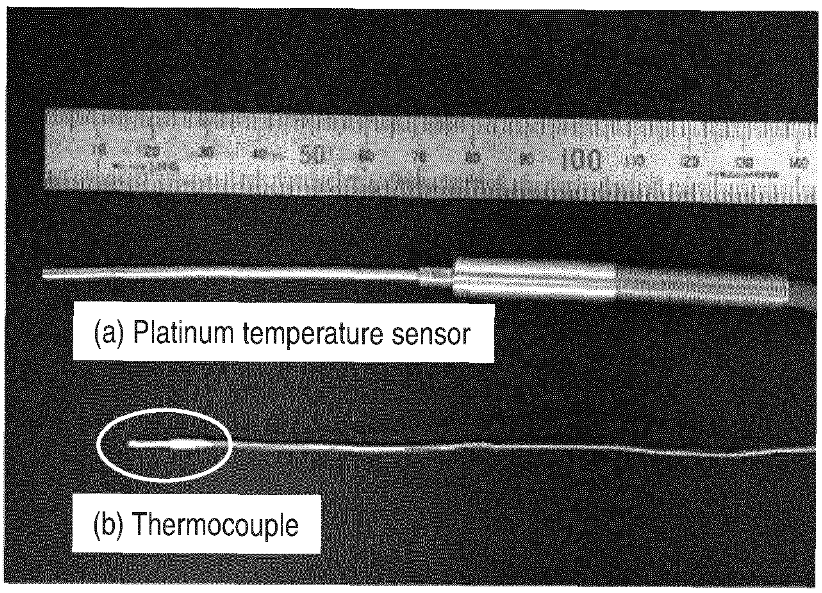
(a) Platinum temperature sensor
(b) Thermocouple

## INTERNATIONAL SEARCH REPORT

International application No.
PCT/JP2018/022781

A. CLASSIFICATION OF SUBJECT MATTER
Int.Cl. G01N27/416(2006.01)i, G01N27/26(2006.01)i, G01N27/28(2006.01)i, G01N27/30(2006.01)i, G01N27/49(2006.01)i

According to International Patent Classification (IPC) or to both national classification and IPC

B. FIELDS SEARCHED

Minimum documentation searched (classification system followed by classification symbols)
Int.Cl. G01N27/416, G01N27/26, G01N27/28, G01N27/30, G01N27/49

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2016-538555 A (ELEMENT SIX TECHNOLOGIES LIMITED) 08 December 2016, claims 8-10, 12, 22-23, 25, paragraphs [0006]-[0007], [0012], [0017]-[0026], fig. 1-7 & US 2016/0282293 A1, paragraphs [0016], [0020], [0043], [0054]-[0078], claims 8-10, 12, 22-23, 25, fig. 1-7 & GB 2520753 A & GB 201321131 D0 & WO 2015/079257 A2 & EP 3074764 A2 | 1-26 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
24 August 2018 (24.08.2018)

Date of mailing of the international search report
04 September 2018 (04.09.2018)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No. PCT/JP2018/022781

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-139725 A (KEIO GIJUKU) 07 June 2007, abstract, paragraphs [0037]-[0049], fig. 4 & US 2007/0114137 A1, paragraphs [0047]-[0058], fig. 4 & EP 1788382 A1 | 1-26 |
| Y | JP 2004-223419 A (FUJI ELECTRIC RETAIL SYSTEMS CO., LTD.) 12 August 2004, paragraphs [0033]-[0034] (Family: none) | 2, 6, 13, 15, 26 |
| Y | JP 2004-525389 A (CSEM CENTRE SUISSE D'ELECTRONIQUE ET DE MICROTECHNIQUE SA) 19 August 2004, paragraphs [0032]-[0033] & US 2004/0154934 A1, paragraphs [0050]-[0051] & WO 2002/095387 A1 & EP 1260813 A1 & CH 697478 B1 | 3-6, 13, 16-19, 26 |
| Y | JP 2014-190660 A (PANASONIC CORP.) 06 October 2014, paragraph [0019] (Family: none) | 7-10 |
| Y | Microfilm of the specification and drawings annexed to the request of Japanese Utility Model Application No. 165432/1978 (Laid-open No. 81760/1980)(HOKUSHIN DENKI SEISAKUSHO) 05 June 1980, specification, page 6, line 11 to page 7, line 1 (Family: none) | 7-10 |
| P, X | JP 2017-133935 A (KEIO GIJUKU) 03 August 2017, entire text, all drawings (Family: none) | 1-26 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 55017939 A
- JP 2007139725 A
- JP 4734097 B
- JP 2003240712 A
- JP 2017118895 A
- JP 2006098281 A
- JP 2011152324 A
- JP 2015172401 A


### Non-patent literature cited in the description


- **MASAKI MATSUO.** Fundamentals and Utilization Technologies of Electrolytic Water. Gihodo Shuppan Co., Ltd, 73
- **MASAKI MATSUO.** Fundamentals and Utilization Technologies of Electrolytic Water. Gihodo Shuppan Co., Ltd, 25 January 2000
- **BARD et al.** L.R., Electrochemical Methods: Fundamentals and Applications. John Wiley & Sons, 2000
- Handbook of Electrochemistry. Elsevier, 2007
- **KISSINGER et al.** Laboratory Techniques in Electroanalytical Chemistry. CRC Press, 1996
- The Acid Ionization Constant of HOCl from 5 to 35. **J. CARRELL MORRIS.** Division of Engineering and Applied Physics. Harvard University, Cambridge, 11 April 1966